# EUROPÄISCHE PATENTSCHRIFT

(11) **EP 1 154 751 B1**
(45) Veröffentlichungstag und Bekanntmachung des Hinweises auf die Patenterteilung: **28.08.2002**
(21) Anmeldenummer: 00906310.8
(22) Anmeldetag: 10.02.2000
(51) Int. Cl.: A61K 7/06

(54) **HAARKOSMETISCHE FORMULIERUNGEN**
FORMULATIONS OF HAIR COSMETICS
PREPARATIONS CAPILLAIRES COSMETIQUES

(30) Priorität: 22.02.1999 DE 19907587
(43) Veröffentlichungstag der Anmeldung: 21.11.2001
(73) Patentinhaber: BASF AKTIENGESELLSCHAFT, 67056 Ludwigshafen (DE)
(72) Erfinder: DIEING, Reinhold, D-67346 Speyer (DE); GOTSCHE, Michael, D-68167 Mannheim (DE); HÖSSEL, Peter, D-67105 Schifferstadt (DE); SANNER, Axel, D-67227 Frankenthal (DE); LEINENBACH, Alfred, D-77731 Willstät (DE); RUTHERFORD, Keith Leslie, Merseyside CH62 5EG (GB)
(86) Internationale Anmeldenummer: EP0001070
(87) Internationale Veröffentlichungsnummer: WO00049998

(56) Entgegenhaltungen:
- WO-A-99/04750
- US-A- 3 946 749
- US-A- 4 876 083
- US-A- 4 880 618

## Beschreibung

Die vorliegende Erfindung betrifft wäßrige oder wäßrig/alkoholische haarkosmetische Formulierungen enthaltend als Filmbildner Polymerisate, hergestellt durch Polymerisation von Vinylestern und optional weiteren radikalisch copolymerisierbaren Monomeren in Gegenwart einer polyetherhaltigen Verbindung, und anschließende zumindest teilweise Verseifung.

Für die Festigung von Haarfrisuren werden seit fast 50 Jahren synthetische Polymere eingesetzt. Während in der Anfangszeit bevorzugt Vinyllactam-Homo- und Copolymere zum Einsatz kamen, haben später carboxylatgruppenhaltige Polymere zunehmend an Bedeutung gewonnen. Anforderungen an Haarfestigerharze sind z.B. eine starke Festigung bei hoher Luftfeuchtigkeit, Elastizität, Auswaschbarkeit vom Haar und Verträglichkeit mit weiteren Formulierungskomponenten. Schwierigkeiten bereitet die Kombination verschiedener Eigenschaften. So zeigen Polymere mit guten Festigungseigenschaften oftmals geringe Elastizitäten, so daß bei mechanischer Beanspruchung der Frisur die Festigungswirkung durch Schädigung des Polymerfilm oft erheblich beeinträchtigt wird.

Verbesserungsbedarf besteht daher vor allem bei der Erzeugung elastischer Frisuren bei gleichzeitig starker Festigung auch bei hoher Luftfeuchtigkeit, guter Auswaschbarkeit und gutem Griff des Haares.

Ziel der Erfindung war es daher, haarkosmetische Formulierungen mit filmbildenden Polymeren zu finden, die der Frisur eine starke Festigung bei gleichzeitig hoher Elastizität verleihen.

Die Aufgabe wurde erfindungsgemäß gelöst durch Verwendung von Polymerisaten, die erhältlich sind durch radikalische Polymerisation von
a) mindestens einem Vinylester in Gegenwart von
b) polyetherhaltigen Verbindungen
und gegebenenfalls mindestens eines weiteren copolymerisierbaren Monomeren c) und anschließender zumindest teilweiser Verseifung der Esterfunktionen der ursprünglichen Monomere a), in haarkosmetischen Formulierungen.

Pfropfpolymerisate von Polyvinylalkohol auf Polyalkylenglykole sind bereits bekannt.

DE 1 077 430 beschreibt ein Verfahren zur Herstellung von Pfropfpolymerisaten von Vinylestern auf Polyalkylenglykole.

DE 1 094 457 und DE 1 081 229 beschreiben Verfahren zur Herstellung von Pfropfpolymerisaten von Polyvinylalkohol auf Polyalkylenglykolen durch Verseifung der Vinylester und deren Verwendung als Schutzkolloide, wasserlösliche Verpackungsfolien, als Schlichte- und Appreturmittel für Textilien und in der Kosmetik.

Bei der Herstellung der erfindungsgemäß verwendeten Polymerisate kann es während der Polymerisation zu einer Pfropfung auf die polyetherhaltigen Verbindungen (b) kommen, was zu den vorteilhaften Eigenschaften der Polymerisate führen kann. Es sind jedoch auch andere Mechanismen als Pfropfung vorstellbar.

Je nach Pfropfungsgrad sind unter den erfindungsgemäß verwendeten Polymerisaten sowohl reine Pfropfpolymerisate als auch Mischungen der o.g. Pfropfpolymerisate mit ungepfropften polyetherhaltigen Verbindungen und Homo- oder Copolymerisaten der Monomeren a) und c) zu verstehen.

Als polyetherhaltige Verbindungen (b) können sowohl Polyalkylenoxide auf Basis von Ethylenoxid, Propylenoxid, Butylenoxid und weiteren Alkylenoxiden als auch Polyglycerin verwendet werden.
Je nach Art der Monomerbausteine enthalten die Polymere folgende Struktureinheiten.
-(CH₂)₂-O-, -(CH₂)₃-O-, -(CH₂)₄-O-, -CH₂-CH(R⁶)-O-, -CH₂-CHOR⁷-CH₂-O-
mit
- R⁶: C₁-C₂₄-Alkyl;
- R⁷: Wasserstoff, C₁-C₂₄-Alkyl, R⁶-C(=O)-, R⁶-NH-C(=O)-.

Dabei kann es sich bei den Struktureinheiten sowohl um Homopolymere als auch um statistische Copolymere und Blockcopolymere handeln.

Bevorzugt werden als Polyether (b) Polymerisate der allgemeinen Formel I verwendet, 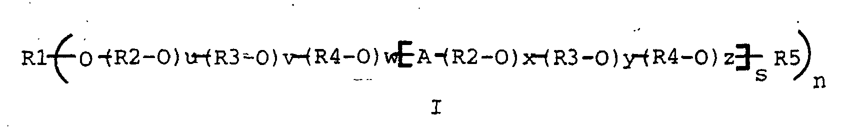 in der die Variablen unabhängig voneinander folgende Bedeutung haben :
- R¹: Wasserstoff, C₁-C₂₄-Alkyl, R⁶-C(=O)-, R⁶-NH-C(=O)-, Polyalkoholrest;
- R⁵: Wasserstoff, C₁-C₂₄-Alkyl, R⁶-C(=O)-, R⁶-NH-C(=O)-;
- R² bis R⁴: -(CH₂)₂-, -(CH₂)₃-, -(CH₂)₄-, -CH₂-CH(R⁶)-, -CH₂-CHOR⁷-CH₂-;
- R⁶: C₁-C₂₄-Alkyl;
- R⁷: Wasserstoff, C₁-C₂₄-Alkyl, R⁶-C(=O)-, R⁶-NH-C(=O)-;
- A: -C(=O)-O, -C(=O)-B-C(=O)-O, -C(=O)-NH-B-NH-C(=O)-O;
- B: -(CH₂)ₜ-, Arylen, ggf. substituiert;
- n: 1 bis 1000;
- s: 0 bis 1000;
- t: 1 bis 12;
- u: 1 bis 5000;
- v: 0 bis 5000;
- w: 0 bis 5000:
- x: 0 bis 5000;
- y: 0 bis 5000;
- z: 0 bis 5000.

Die endständigen primären Hydroxylgruppen der auf Basis von Polyalkylenoxiden hergestellten Polyether sowie die sekundären OH-Gruppen von Polyglycerin können dabei sowohl in ungeschützter Form frei vorliegen als auch mit Alkoholen einer Kettenlänge C₁-C₂₄ bzw. mit Carbonsäuren einer Kettenlänge C₁-C₂₄ verethert bzw. verestert werden oder mit Isocyanaten zu Urethanen umgesetzt werden.

Als Alkylreste für R¹ und R⁵ bis R⁷ seien verzweigte oder unverzweigte C₁-C₂₄-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethylhexyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als bevorzugte Vertreter der oben genannten Alkylreste seien verzweigte oder unverzweigte C₁-C₁₂-, besonders bevorzugt C₁-C₆-Alkylketten genannt.

Das Molekulargewicht der Polyether liegt im Bereich kleiner 1000000 (nach Zahlenmittel), bevorzugt im Bereich von 300 bis 100000, besonders bevorzugt im Bereich von 500 bis 50000, ganz besonders bevorzugt im Bereich von 800 bis 40000.

Vorteilhafterweise verwendet man Homopolymerisate des Ethylenoxids oder Copolymerisate, mit einem Ethylenoxidanteil von 40 bis 99 Gew.-%. Für die bevorzugt einzusetzenden Ethylenoxidpolymerisate beträgt somit der Anteil an einpolymerisiertem Ethylenoxid 40 bis 100 mol-%. Als Comonomer für diese Copolymerisate kommen Propylenoxid, Butylenoxid und/oder Isobutylenoxid in -Betracht. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Der Ethylenoxidanteil der Copolymerisate beträgt vorzugsweise 40 bis 99 mol-%, der Propylenoxidanteil 1 bis 60 mol-% und der Anteil an Butylenoxid in den Copolymerisaten 1 bis 30 mol-%. Neben geradkettigen können auch verzweigte Homo- oder Copolymerisate als polyetherhaltige Verbindungen b) verwendet werden.

Verzweigte Polymerisate können hergestellt werden, indem man beispielsweise an Polyalkoholresten, z.B. an Pentaerythrit, Glycerin oder an Zuckeralkoholen wie D-Sorbit und D-Marinit aber auch an Polysaccharide wie Cellulose und Stärke, Ethylenoxid und gegebenenfalls noch Propylenoxid und/oder Butylenoxide anlagert. .Die Alkylenoxid-Einheiten können im Polymerisat statistisch verteilt sein oder in Form von Blöcken vorliegen.

Es ist aber auch möglich, Polyester von Polyalkylenoxiden und aliphatischen oder aromatischen Dicarbonsäuren, z.B. Oxalsäure, Bernsteinsäure, Adipinsäure und Terephthalsäure mit Molmassen von 1500 bis 25000, wie z.B. beschrieben in EP-A-0 743 962, als polyetherhaltige Verbindung zu verwenden. Des weiteren können auch Polycarbonate durch Umsetzung von Polyalkylenoxiden mit Phosgen oder Carbonaten wie z.B. Diphenylcarbonat, sowie Polyurethane durch Umsetzung von Polyalkylenoxiden mit aliphatischen und aromatischen Diisocyanaten verwendet werden.

Besonders bevorzugt werden als Polyether (b) Polymerisate der allgemeinen Formel I mit einem mittleren Molekulargewicht von 300 bis 100.000 (nach dem Zahlenmittel), in der die Variablen unabhängig voneinander folgende Bedeutung haben:
- R¹: Wasserstoff, C₁-C1₂-Alkyl, R⁶-C(=O)-, R⁶-NH-C(=O)-, Polyalkoholrest;
- R⁵: Wasserstoff, C₁-C₁₂-Alkyl, R⁶-C(=O)-, R⁶-NH-C(=O)-;
- R² bis R⁴: -(CH₂)₂-, -(CH₂)₃-, -(CH₂)₄-, -CH₂-CH(R⁶)-, -CH₂-CHOR⁷-CH₂-;
- R⁶: C₁-C₁₂-Alkyl;
- R⁷: Wasserstoff, C₁-C₁₂-Alkyl, R⁶-C(=O)-, R⁶-NH-C(=O)-;
- n: 1 bis 8;
- s: 0;
- u: 2 bis 2000;
- v: 0 bis 2000;
- w: 0 bis 2000.

Ganz besonders bevorzugt werden als Polyether b) Polymerisate der allgemeinen Formel I mit einem mittleren Molekulargewicht von 500 bis 50000 (nach dem Zahlenmittel), in der die Variablen unabhängig voneinander folgende Bedeutung haben:
- R¹: Wasserstoff, C₁-C₆-Alkyl, R⁶-C(=O)-, R⁶-NH-C(=O)-;
- R⁵: Wasserstoff, C₁-C₆-Alkyl, R⁶-C(=O)-, R⁶-NH-C(=O)-;
- R² bis R⁴: -(CH₂)₂-, -(CH₂)₃-, -(CH₂)₄-, -CH₂-CH(R⁶)-, -CH₂-CHOR⁷-CH₂-;
- R⁶: C₁-C₆-Alkyl;
- R⁷: Wasserstoff, C₁-C₆-Alkyl, R⁶-C(=O)-, R⁶-NH-C(=O)-;
- n: 1;
- s: 0;
- u: 5 bis 500;
- v: 0 bis 500;
- w: 0 bis 500.

Als Polyether können jedoch auch Silikonderivate eingesetzt werden. Geeignete Silikonderivate sind die unter dem INCI Namen Dimethicone Copolyole oder Silikontenside bekannten Verbindungen wie zum Beispiel die unter den Markennamen Abil® (der Fa. T. Goldschmidt), Alkasil® (der Fa. Rhône-Poulenc), Silicone Polyol Copolymer® (der Fa Genesee), Belsil® (der Fa. Wacker), Silwet® (der Fa. Witco, Greenwich, CT, USA) oder Dow Corning (der Fa. Dow Corning) erhältlich. Diese beinhalten Verbindungen mit den CAS-Nummern 64365-23-7; 68937-54-2; 68938-54-5; 68937-55-3.

Silikone werden in der Haarkosmetik im allgemeinen zur Verbesserung des Griffs eingesetzt. Die Verwendung von polyetherhaltigen Silikonderivaten als Polyether (b) in den erfindungsgemäßen Polymeren kann deshalb zusätzlich zu einer Verbesserung des Griffs der Haare führen.

Bevorzugte Vertreter solcher polyetherhaltigen Silikonderivaten sind solche, die die folgenden Strukturelemente enthalten: 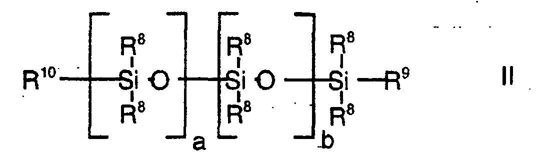 wobei:
R⁹ = CH₃ oder 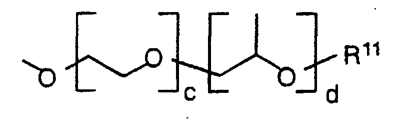
R¹⁰ = CH₃ oder R⁹
R¹¹ = H, CH₃,

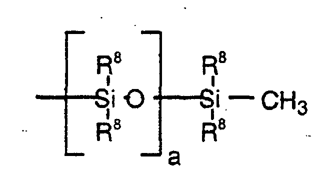

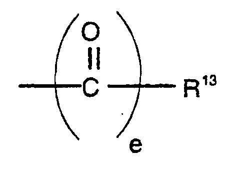

R¹³ ein organischer Rest aus 1 bis 40 Kohlenstoffatomen, der Amino-, Carbonsäure- oder Sulfonatgruppen enthalten kann oder für den Fall e=0, auch das Anion einer anorganischen Säure bedeutet,
und wobei die Reste R⁸ identisch oder unterschiedlich sein können, und entweder aus der Gruppe der aliphatischen Kohlenwasserstoffe mit 1 bis 20 Kohlenstoffatomen stammen, cyclische aliphatische Kohlenwasserstoffe mit 3 bis 20 C-Atomen sind, aromatischer Natur oder gleich R¹² sind, wobei:
R¹² = 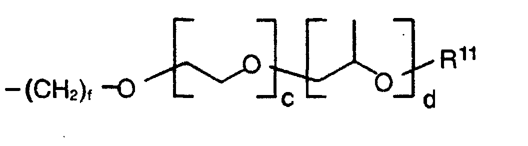 mit der Maßgabe, daß mindestens einer der Reste R⁸, R⁹ oder R¹⁰ ein polyalkylenoxidhaltiger Rest nach obengenannter Definition ist,
   und f eine ganze Zahl von 1 bis 6 ist,
   a und b ganze Zahlen derart sind, daß das Molekulargewicht des Polysiloxan-Blocks zwischen 300 und 30000 liegt,
   c und d ganze Zahlen zwischen 0 und 50 sein können mit der Maßgabe, daß die Summe aus c und d größer als 0 ist, und e 0 oder 1 ist.

Bevorzugte Reste R⁹ und R¹² sind solche, bei denen die Summe aus c+d zwischen 5 und 30 beträgt.

Bevorzugt werden die Gruppen R⁸ aus der folgenden Gruppe ausgewählt: Methyl, Ethyl, Propyl, Butyl, Isobutyl; Pentyl, Isopentyl, Hexyl, Octyl, Decyl, Dodecyl und Octadecyl, cycloaliphatische Reste, speziell Cyclohexyl, aromatische Gruppen, speziell Phenyl oder Naphthyl, gemischt aromatisch-aliphatische Reste wie Benzyl oder Phenylethyl sowie Tolyl und Xylyl und R¹².

Besonders geeignete Reste R¹¹ sind solche, bei denen im Falle von R¹¹ = -(CO)ₑ-R¹³ R¹³ ein beliebiger Alkyl-, Cycloalkyl oder Arylrest bedeutet, der zwischen 1 und 40 C-Atomen besitzt und der weitere ionogene Gruppen wie NH₂, COOH, SO₃H tragen kann.

Bevorzugte anorganische Reste R¹³ sind, für den Fall e=0, Phosphat und Sulfat.

Besonders bevorzugte polyetherhaltige Silikonderivate sind solche der allgemeinen Struktur:

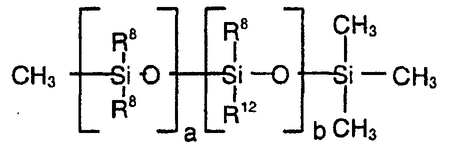

Des weiteren können als Polyether (b) auch Homo- und Copolymerisate aus polyalkylenoxidhaltigen ethylenisch ungesättigten Monomeren wie beispielsweise Polyalkylenoxid(meth)acrylate, Polyalkylenoxidvinylether, Polyalkylenoxid(meth)acrylamide, Polyalkylenoxidallyamide oder Polyalkylenoxidvinylamide verwendet werden. Selbstverständlich können auch Copolymerisate solcher Monomere mit anderen ethylenisch ungesättigten Monomeren eingesetzt werden.

Als polyetherhaltige Verbindungen b) können aber auch Umsetzungsprodukte von Polyethyleniminen mit Akylenoxiden eingesetzt werden. Als Alkylenoxide werden in diesem Fall bevorzugt Ethylenoxid, Propylenoxid, Butylenoxid und Mischungen aus diesen, besonders bevorzugt Ethylenoxid verwendet. Als Polyethylenimine können Polymere mit zahlenmittleren Molekulargewichten von 300 bis 20000, bevorzugt 500 bis 10000, ganz besonders bevorzugt 500 bis 5000, eingesetzt werden. Das Gewichtsverhältnis zwischen eingesetztem Alkylenoxid und Polyethylenimin liegt im Bereich von 100 : 1 bis 0,1 : 1, bevorzugt im Bereich 50 : 1 bis 0,5 : 1, ganz besonders bevorzugt im Bereich 20 : 1 bis 0,5 : 1.

Für die Polymerisation in Gegenwart der Polyether b) seien als Komponente a) folgende radikalisch polymerisierbare Monomere genannt:
Vinylester von aliphatischen, gesättigten oder ungesättigten C₁-C₂₄-Carbonsäuren, wie z.B. Ameisensäure, Essigsäure, Propionsäure, Buttersäure, Valeriansäure, Isovaleriansäure, Capronsäure, Caprylsäure, Caprinsäure, Undecylensäure, Laurinsäure, Myristinsäure, Palmitinsäure, Palmitoleinsäure, Stearinsäure, Ölsäure, Arachinsäure, Behensäure, Lignocerinsäure, Cerotinsäure sowie Melissensäure.

Bevorzugt werden Vinylester der oben genannten C₁-C₁₂-Carbonsäuren, insbesondere der C₁-C₆-Carbonsäuren, verwendet. Ganz besonders bevorzugt ist Vinylacetat.

Selbstverständlich können auch Mischungen der jeweiligen Monomeren aus der Gruppe a) copolymerisiert werden.

Die Vinylester (a) können daneben auch in Mischung mit einem oder mehreren, ethylenisch ungesättigten copolymerisierbaren Comonomeren (c) eingesetzt werden, wobei der Anteil dieser zusätzlichen Monomeren auf maximal 50 Gew.-% beschränkt sein sollte. Bevorzugt sind Anteile von 0 bis 20 Gew.-%. Der Begriff ethylenisch ungesättigt bedeutet, daß die Monomere zumindest eine radikalisch polymerisierbare Kohlenstoff-Kohlenstoff Doppelbindung besitzen, die mono-, di-, tri- oder tetrasubstituiert sein kann.

Die bevorzugten zusätzlich eingesetzten ethylenisch ungesättigten Comonomere (c) können durch die folgende allgemeine Formel beschrieben werden:

X-C(O)CR¹⁵=CHR¹⁴

wobei
X ausgewählt ist aus der Gruppe der Reste -OH, -OM, -OR¹⁶, NH₂, -NHR¹⁶, N(R¹⁶)₂ ;
M ist ein Kation ausgewählt aus der Gruppe bestehend aus: Na⁺, K⁺, Mg⁺⁺, Ca⁺⁺, Zn⁺⁺, NH₄⁺, Alkylammonium, Dialkylammonium, Trialkylammonium und Tetraalkylammonium;
die Reste R¹⁶ können identisch oder verschieden ausgewählt werden aus der Gruppe bestehend aus -H, C₁-C₄₀ linear- oder verzweigtkettige Alkylreste, N,N-Dimethylaminoethyl, 2-Hydroxyethyl, 2-Methoxyethyl, 2-Ethoxyethyl, Hydroxypropyl, Methoxypropyl oder Ethoxypropyl.

R¹⁵ und R¹⁴ sind unabhängig voneinander ausgewählt aus der Gruppe bestehend aus: -H, C₁-C₈ linear- oder verzweigtkettige Alkylketten, Methoxy, Ethoxy, 2-Hydroxyethoxy, 2-Methoxyethoxy und 2-Ethoxyethyl.

Repräsentative aber nicht limitierende Beispiele von geeigneten Monomeren (c) sind zum Beispiel Acrylsäure oder Methacrylsäure und deren Salze, Ester und Amide. Die Salze können von jedem beliebigen nicht toxischen Metall, Ammonium oder substituierten Ammonium-Gegenionen abgeleitet sein.

Die Ester können abgeleitet sein von C₁-C₄₀ linearen, C₃-C₄₀ verzweigtkettigen oder C₃-C₄₀ carbocyclischen Alkoholen, von mehrfachfunktionellen Alkoholen mit 2 bis etwa 8 Hydroxylgruppen wie Ethylenglycol, Hexylenglycol, Glycerin und 1,2,6-Hexantriol, von Aminoalkoholen oder von Alkoholethern wie Methoxyethanol und Ethoxyethanol, (Alkyl)Polyethylenglykolen, (Alkly)Polypropylenglykolen oder ethoxylierten Fettalkoholen, beispielsweise C₁₂-C₂₄-Fettalkoholen umgesetzt mit 1 bis 200 Ethylenoxid-Einheiten.

Ferner eignen sich N,N-Dialkylaminoalkylacrylate- und -methacrylate und N-Dialkylaminoalkylacryl- und -methacrylamide der allgemeinen Formel (III) 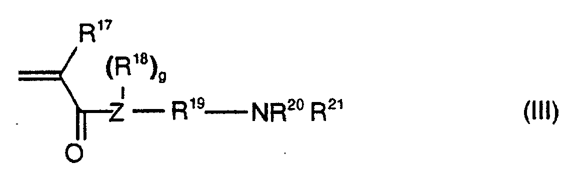 mit
- R¹⁷ =: H, Alkyl mit 1 bis 8 C-Atomen,
- R¹⁸ =: H, Methyl,
- R¹⁹ =: Alkylen mit 1 bis 24 C-Atomen, optional substituiert durch Alkyl,
- R²⁰, R²¹ =: C₁-C₄₀ Alkylrest,
- Z =: Stickstoff für g = 1 oder Sauerstoff für g = 0

Die Amide können unsubstituiert, N-Alkyl oder N-Alkylamino monosubstituiert oder N,N-dialkylsubstituiert oder N,N-dialkylaminodisubstituiert vorliegen, worin die Alkyl- oder Alkylaminogruppen von C₁-C₄₀ linearen, C₃-C₄₀ verzweigtkettigen, oder C₃-C₄₀ carbocyclischen Einheiten abgeleitet sind. Zusätzlich können die Alkylaminogruppen quaternisiert werden.

Bevorzugte Comonomere der Formel III sind N,N-Dimethylaminomethyl(meth)acrylat, N,N-Diethylaminomethyl(meth)acrylat, N,N-Dimethylaminoethyl(meth)acrylat, N,N-Diethylaminoethyl(meth)acrylat, N-[3-(dimethylamino)propyl]methacrylamid und N-[3-(dimethylamino)propyl]acrylamid.

Ebenfalls verwendbare Comonomere (c) sind substituierte Acrylsäuren sowie Salze, Ester und Amide davon, wobei die Substituenten an den Kohlenstoffatomen in der zwei oder drei Position der Acrylsäure stehen, und unabhängig voneinander ausgewählt sind aus der Gruppe bestehend aus C₁-C₄ Alkyl, -CN, COOH besonders bevorzugt Methacrylsäure, Ethacrylsäure und 3-Cyanoacrylsäure. Diese Salze, Ester und Amide dieser substituierten Acrylsäuren können wie oben für die Salze, Ester und Amide der Acrylsäure beschrieben ausgewählt werden.

Andere geeignete Comonomere (c) sind Allylester von C₁-C₄₀ linearen, C₃-C₄₀ verzweigtkettigen oder C₃-C₄₀ carbocyclische Carbonsäuren, Vinyl- oder Allylhalogenide, bevorzugt Vinylchlorid und Allylchlorid, Vinylether, bevorzugt Methyl-, Ethyl-, Butyl- oder Dodecylvinylether, Vinylformamid, Vinylmethylacetamid, Vinylamin; Vinyllactame, bevorzugt Vinylpyrrolidon und Vinylcaprolactam, Vinyl- oder Allyl-substituierte heterocyclische Verbindungen, bevorzugt Vinylpyridin, Vinyloxazolin und Allylpyridin.

Weiterhin sind N-Vinylimidazole der allgemeinen Formel IV geeignet, worin R²² bis R²⁴ unabhängig voneinander für Wasserstoff, C₁-C₄-Alkyl oder Phenyl steht:

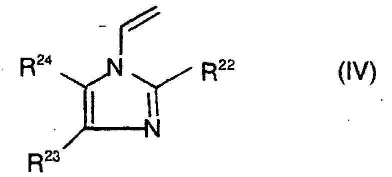

Weitere geeignete Comonomere (c) sind Diallylamine der allgemeinen Formel (V) 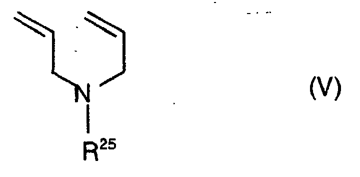 mit R²⁵ = C₁- bis C₂₄-Alkyl

Weitere geeignete Comonomere (c) sind Vinylidenchlorid; und Kohlenwasserstoffe mit mindestens einer Kohlenstoff-Kohlenstoff Doppelbindung, bevorzugt Styrol, alpha-Methylstyrol, tert.-Butylstyrol, Butadien, Isopren, Cyclohexadien, Ethylen, Propylen, 1-Buten, 2-Buten, Isobutylen, Vinyltoluol, sowie Mischungen dieser Monomere.

Besonders geeignete Comonomere (c) sind Acrylsäure, Methacrylsäure, Ethylacrylsäure, Methylacrylat, Ethylacrylat, Propylacrylat, n-Butylacrylat, iso-Butylacrylat, t-Butylacrylat, 2-Ethylhexylacrylat, Decylacrylat, Methylmethacrylat, Ethylmethacrylat, Propylmethacrylat, n-Butylmethacrylat, iso-Butylmethacrylat, t-Butylmethacrylat, 2-Ethylhexylmethacrylat, Decylmethacrylat, Methylethacrylat, Ethylethacrylat, n-Butylethacrylat, iso-Butylethacrylat, t-Butyl-ethacrylat, 2-Ethylhexylethacrylat, Decylethacrylat, Stearyl(meth)acrylat, 2,3-Dihydroxypropylacrylat, 2,3-Dihydroxypropylmethacrylat, 2-Hydroxyethylacrylat, Hydroxypropylacrylate, 2-Hydroxyethylmethacrylat, 2-Hydroxyethylethacrylat, 2-Methoxyethylacrylat, 2-Methoxyethylmethacrylat, 2-Methoxyethylethacrylat, 2-Ethoxyethylmethacrylat, 2-Ethoxyethylethacrylat, Hydroxypropylmethacrylate, Glycerylmonoacrylat, Glycerylmonomethacrylat, Polyalkylenglykol(meth)acrylate, ungesättigte Sulfonsäuren wie zum Beispiel Acrylamidopropansulfonsäure;
Acrylamid, Methacrylamid, Ethacrylamid, N-Methylacrylamid, N,N-Dimethylacrylamid, N-Ethylacrylamid, N-Isopropylacrylamid, N-Butylacrylamid, N-t-Butylacrylamid, N-Octylacrylamid, N-t-Octylacrylamid, N-Octadecylacrylamid, N-Phenylacrylamid, N-Methylmethacrylamid, N-Ethylmethacrylamid, N-Dodecylmethacrylamid, 1-Vinylimidazol, 1-Vinyl-2-methylvinylimidazol, N,N-Dimethylaminomethyl(meth)acrylat, N,N-Diethylaminomethyl(meth)-acrylat, N,N-Dimethylaminoethyl(meth)acrylat, N,N-Diethylaminoethyl(meth)acrylat, N,N-Dimethylaminobutyl(meth)acrylat, N,N-Diethylaminobutyl(meth)acrylat, N,N-Dimethylaminohexyl(meth)-acrylat, N,N-Dimethylaminooctyl(meth)acrylat, N,N-Dimethylaminododecyl(meth)acrylat, N-[3-(dimethylamino)propyl]methacrylamid, N-[3-(dimethylamino)propyl]acrylamid, N-[3-(dimethylamino)-butyl]methacrylamid, N-[8-(dimethylamino)octyl]methacrylamid, N- [12-(dimethylamino)dodecyl]methacrylamid, N-[3-(diethylamino)-propyl]methacrylamid, N-[3-(diethylamino)propyl]acrylamid;

Maleinsäure, Fumarsäure, Maleinsäureanhydrid und seine Halbester, Crotonsäure, Itaconsäure, Diallyldimethylammoniumchlorid, Vinylether (zum Beispiel: Methyl-, Ethyl-, Butyl- oder Dodecylvinylether), Vinylformamid, Vinylmethylacetamid, Vinylamin; Methylvinylketon, Maleimid, Vinylpyridin, Vinylimidazol, Vinylfuran, Styrol, Styrolsulfonat, Allylalkohol, und Mischungen daraus.

Von diesen sind besonders bevorzugt Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, Stearylacrylat, Stearylmethacrylat, N-t-Butylacrylamid, N-Octylacrylamid, 2-Hydroxyethylacrylat, Hydroxypropylacrylate, 2-Hydroxyethylmethacrylat, Hydroxypropylmethacrylate, Alkylenglykol(meth)acrylate, Styrol, ungesättigte Sulfonsäuren wie zum Beispiel Acrylamidopropansulfonsäure, Vinylpyrrolidon, Vinylcaprolactam, Vinylether (z.B.: Methyl-, Ethyl-, Butyl- oder Dodecylvinylether), Vinylformamid, Vinylmethylacetamid, Vinylamin, 1-Vinylimidazol, 1-Vinyl-2-methylimidazol, N,N-Dimethylaminomethylmethacrylat und N- [3- (dimethylamino)-propyl]methacrylamid; 3-Methyl-1-vinylimidazoliumchlorid, 3-Methyl-1-vinylimidazoliummethylsulfat, N,N-Dimethylaminoethylmethacrylat, N-[3-(dimethylamino)propyl]methacrylamid quaternisiert mit Methylchlorid, Methylsulfat oder Diethylsulfat.

Monomere, mit einem basischen Stickstoffatom, können dabei auf folgende Weise quarternisiert werden: Zur Quaternisierung der Amine eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternierung der basischen Amine kann auch mit Alkylenoxiden wie Ethylenoxid oder Propylenoxid in Gegenwart von Säuren durchgeführt werden. Bevorzugte Quaternierungsmittel sind: Methylchlorid, Dimethylsulfat oder Diethylsulfat.

Die Quaternisierung kann vor der Polymerisation oder nach der Polymerisation durchgeführt werden.

Außerdem können die Umsetzungsprodukte von ungesättigten Säuren, wie z.B. Acrylsäure oder Methacrylsäure, mit einem quaternisierten Epichlorhydrin der allgemeinen Formel (VI) eingesetzt werden (R²⁶ = C₁- bis C₄₀-Alkyl). 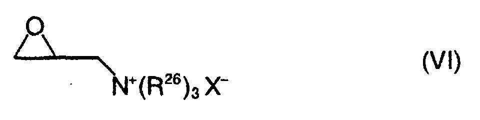 Beispiele hierfür sind zum Beispiel:
(Meth)acryloyloxyhydroxypropyltrimethylammoniumchlorid und
(Meth)acryloyloxyhydroxypropyltriethylammoniumchlorid.

Die basischen Monomere können auch kationisiert werden, indem sie mit Mineralsäuren, wie z.B. Schwefelsäure, Chlorwasserstoffsäure, Bromwasserstoffsäure, Iodwasserstoffsäure, Phosphorsäure oder Salpetersäure, oder mit organischen Säuren, wie z.B. Ameisensäure, Essigsäure, Milchsäure, oder Citronensäure, neutralisiert werden.

Zusätzlich zu den oben genannten Comonomeren können als Comonomere (c) sogenannte Makromonomere wie zum Beispiel silikonhaltige Makromonomere mit ein oder mehreren radikalisch polymerisierbaren Gruppen oder Alkyloxazolinmakromonomere eingesetzt werden, wie sie zum Beispiel in der EP 408 311 beschrieben sind.

Des weiteren können fluorhaltige Monomere, wie sie beispielsweise in der EP 558423 beschrieben sind, vernetzend wirkende oder das Molekulargewicht regelnde Verbindungen in Kombination oder alleine eingesetzt werden.

Als Regler können die üblichen dem Fachmann bekannten Verbindungen, wie zum Beispiel Schwefelverbindungen (z.B.: Mercaptoethanol, 2-Ethylhexylthioglykolat, Thioglykolsäure oder Dodecylmercaptan), sowie Tribromchlormethan oder andere Verbindungen, die regelnd auf das Molekulargewicht der erhaltenen Polymerisate wirken, verwendet werden.

Es können gegebenenfalls auch thiolgruppenhaltige Silikonverbindungen eingesetzt werden.
Bevorzugt werden silikonfreie Regler eingesetzt.

Als vernetzende Monomere können Verbindungen mit mindestens zwei ethylenisch ungesättigten Doppelbindungen eingesetzt werden, wie zum Beispiel Ester von ethylenisch ungesättigten Carbonsäuren, wie Acrylsäure oder Methacrylsäure und mehrwertigen Alkoholen, Ether von mindestens zweiwertigen Alkoholen, wie zum Beispiel Vinylether oder Allylether.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-cyclohexandiol, 1,4-Bis(hydroxymethyl)cyclohexan, Hydroxypivalinsäure-neopentylglycolmonoester, 2,2-Bis(4-hydroxyphenyl)-propan, 2,2-Bis [4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10 000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2, 5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C3- bis C6-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexen oder Polybutadiene mit Molekulargewichten von 200.bis 20000.

Ferner geeignet sind Amide von ungesättigten Carbonsäuren, wie z.B. Acryl- und Methacrylsäure, Itaconsäure, Maleinsäure und N-Allylaminen von mindestens zweiwertigen Aminen, wie z.B. 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin oder entsprechende Ammoniumsalze, z.B. Triallylmethylammoniumchlorid oder -methylsulfat, als Vernetzer geeignet.

Weiterhin können N-Vinylverbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff eingesetzt werden.

Weitere geeignete Vernetzer sind Divinyldioxan, Tetraallylsilan oder Tetravinylsilan.

Besonders bevorzugte Vernetzer sind beispielsweise Methylenbisacrylamid, Divinylbenzol, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind, sowie Allyl- oder Vinylether von mehrwertigen Alkoholen, beispielsweise 1,2-Ethandiol, 1,4-Butandiol, Diethylenglykol, Trimethylolpropan, Glycerin, Pentaerythrit, Sorbitan und Zucker wie Saccharose, Glucose, Mannose.

Ganz besonders bevorzugt als Vernetzer sind Pentaerythrittriallylether, Allylether von Zuckern wie Saccharose, Glucose, Mannose, Divinylbenzol, Methylenbisacrylamid, N,N'-Divinylethylenharnstoff, und (Meth-)Acrylsäureester von Glykol, Butandiol, Trimethylolpropan oder Glycerin oder (Meth)Acrylsäureester von mit Ethylenoxid und/oder Epichlorhydrin umgesetzten Glykol, Butandiol, Trimethylolpropan oder Glycerin.

Der Anteil der vernetzend wirkenden Monomeren beträgt 0 bis 10 Gew.-%, bevorzugt 0,1 bis 5 Gew.-%, ganz besonders bevorzugt 0,2 bis 2 Gew.-%.

Bei der Polymerisation zur Herstellung der erfindungsgemäßen Polymerisate können gegebenenfalls auch andere Polymere, wie zum Beispiel Polyamide, Polyurethane, Polyester, Homo- und Copolymere von ethylenisch ungesättigten Monomeren, zugegen sein. Beispiele für solche zum Teil auch in der Kosmetik eingesetzten Polymeren sind die unter den Handelsnamen bekannten Polymere Amerhold™, Ultrahold™, Ultrahold Strong™, Luviflex™ VBM, Luvimer™, Acronal™, Acudyne™, Stepanhold™, Lovocryl™, Versatyl™, Amphomer™ oder Eastma AQ™.

Die erfindungsgemäßen Comonomere (c) können, sofern sie ionisierbare Gruppen enthalten, vor oder nach der Polymerisation, zum Teil oder vollständig mit Säuren oder Basen neutralisiert werden, um so zum Beispiel die Wasserlöslichkeit oder -dispergierbarkeit auf ein gewünschtes Maß einzustellen.

Als Neutralisationsmittel für Säuregruppen tragende Monomere können zum Beispiel Mineralbasen wie Natriumcarbonat, Alkalihydroxide sowie Ammoniak, organische Basen wie Aminoalkohole speziell 2-Amino-2-Methyl-1-Propanol, Monoethanolamin, Diethanolamin, Triethanolamin, Triisopropanolamin, Tri[(2-hydroxy)1-Propyl]amin, 2-Amino-2-Methyl-1,3-Propandiol, 2-Amino-2-hydroxymethyl-1,3-Propandiol sowie Diamine, wie zum Beispiel Lysin, verwendet werden.

Zur Herstellung der Polymerisate können die Monomeren der Komponente a) in Gegenwart der Polyether sowohl mit Hilfe von Radikale bildenden Initiatoren als auch durch Einwirkung energiereicher Strahlung, worunter auch die Einwirkung energiereicher Elektronen verstanden werden soll, polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, Azobis-(2-amidonopropan)dihydrochlorid oder 2-2'-Azo-bis-(2-methylbutyronitril). Geeignet sind auch Initiatormischungen oder Redox-Initiator-Systeme, wie z.B. Ascorbinsäure/Eisen(II)sulfat/-Natriumperoxodisulfat, tert.-Butylhydroperoxid/Natriumdisulfit, tert.-Butylhydroperoxid/Natriumhydroxymethansulfinat. Bevorzugt werden organische Peroxide eingesetzt.

Die verwendeten Mengen an Initiator bzw. Initiatorgemischen bezogen auf eingesetztes Monomer liegen zwischen 0,01 und 10 Gew.-%, vorzugsweise zwischen 0,1 und 5 Gew.-%.

Die Polymerisation erfolgt im Temperaturbereich von 40 bis 200°C, bevorzugt im Bereich von 50 bis 140°C, besonders bevorzugt im Bereich von 60 bis 110°C. Sie wird üblicherweise unter atmosphärischem Druck durchgeführt, kann jedoch auch unter vermindertem oder erhöhtem Druck, vorzugsweise zwischen 1 und 5 bar, ablaufen.

Die Polymerisation kann beispielsweise als Lösungspolymerisation, Polymerisation in Substanz, Emulsionspolymerisation, umgekehrte Emulsionspolymerisation, Suspensionspolymerisation, umgekehrte Suspensionspolymerisation oder Fällungspolymerisation durchgeführt werden, ohne daß die verwendbaren Methoden darauf beschränkt sind.

Bei der Polymerisation in Substanz kann man so vorgehen, daß man die polyetherhaltige Verbindung b) in mindestens einem Monomer der Gruppe a) und eventuell weiteren Comonomeren der Gruppe c) löst und nach Zugabe eines Polymerisationsinitiators die Mischung auspolymerisiert. Die Polymerisation kann auch halbkontinuierlich durchgeführt werden, indem man zunächst einen Teil, z.B. 10 % des zu polymerisierenden Gemisches aus der polyetherhaltigen Verbindung b), mindestens einem Monomeren der Gruppe a), eventuell weiteren Comonomeren der Gruppe c) und Initiator vorlegt, das Gemisch auf Polymerisationstemperatur erhitzt und nach dem Anspringen der Polymerisation den Rest der zu polymerisierenden Mischung nach Fortschritt der Polymerisation zugibt. Die Polymerisate können auch dadurch erhalten werden, daß man die polyetherhaltigen Verbindungen der Gruppe b) in einem Reaktor vorlegt, auf die Polymerisationstemperatur erwärmt und mindestens ein Monomer der Gruppe a), eventuell weiteren Comonomeren der Gruppe c) und Polymerisationsinitiator entweder auf einmal, absatzweise oder vorzugsweise kontinuierlich zufügt und polymerisiert.

Falls gewünscht, kann die oben beschriebene Polymerisation auch in einem Lösemittel durchgeführt werden. Geeignete Lösemittel sind beispielsweise Alkohole, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Glycerin und Dioxan. Die Polymerisation kann auch in wasser als Lösemittel durchgeführt werden. In diesem Fall liegt zunächst eine Lösung vor, die in Abhängigkeit von der Menge der zugegebenen Monomeren der Komponente a) in Wasser mehr oder weniger gut löslich ist. Um wasserunlösliche Produkte, die während der Polymerisation entstehen können, in Lösung zu überführen, kann man beispielsweise organische Lösemittel zusetzen, wie einwertige Alkohole mit 1 bis 3 Kohlenstoffatomen, Aceton oder Dimethylformamid. Man kann jedoch auch bei der Polymerisation in Wasser so verfahren, daß man die wasserunlöslichen Polymerisate durch Zugabe üblicher Emulgatoren oder Schutzkolloide, z.B. Polyvinylalkohol, in eine feinteilige Dispersion überführt.

Als Emulgatoren verwendet man beispielsweise ionische oder nichtionische Tenside, deren HLB-Wert im Bereich von 3 bis 13 liegt. Zur Definition des HLB-Werts wird auf die Veröffentlichung von W.C. Griffin, J. Soc. Cosmetic Chem., Band 5, 249 (1954), hingewiesen.

Die Menge an Tensiden, bezogen auf das Polymerisat, beträgt 0,1 bis 10 Gew.-%. Bei Verwendung von Wasser als Lösemittel erhält man Lösungen bzw. Dispersionen der Polymerisate. Sofern man Lösungen des Polymerisates in einem organischen Lösemittel herstellt bzw. in Mischungen aus einem organischen Lösemittel und Wasser, so verwendet man pro 100 Gew.-Teile des Polymerisates 5 bis 2000, vorzugsweise 10 bis 500 Gew.-Teile des organischen Lösemittels oder des Lösemittelgemisches.

Bevorzugt sind Polymere, die erhältlich sind durch radikalische Polymerisation von
a) 10 - 98 Gew.-% mindestens eines Vinylesters von C₁-C₂₄-Carbonsäuren in Gegenwart von
b) 2 - 90 Gew.-% mindestens einer polyetherhaltigen Verbindung und
c) 0 - 50 Gew.-% eines oder mehreren weiteren copolymerisierbaren Monomeren

Besonders bevorzugt sind Polymere, die erhältlich sind durch radikalische Polymerisation von
a) 50 - 97 Gew.-% mindestens eines Vinylesters von C₁-C₂₄-Carbonsäuren in Gegenwart von
b) 3 - 50 Gew.-% mindestens einer polyetherhaltigen Verbindung und
c) 0 - 30 Gew.-% eines oder mehreren weiteren copolymerisierbaren Monomeren

Ganz besonders bevorzugt sind Polymere, die erhältlich sind durch radikalische Polymerisation von
a) 60 - 97 Gew.% mindestens eines Vinylesters von C₁-C₂₄-Carbonsäuren in Gegenwart von
b) 3 - 40 Gew.% mindestens einer polyetherhaltigen Verbindung und
c) 0 - 20 Gew.% eines oder mehreren weiteren copolymerisierbaren Monomeren

Zur Herstellung der erfindungsgemäß verwendeten Polymeren werden die Estergruppen der ursprünglichen Monomere a) und gegebenenfalls weiterer Monomere nach der Polymerisation durch Hydrolyse, Alkoholyse oder Aminolyse gespalten. Im nachfolgenden wird dieser Verfahrensschritt allgemein als Verseifung bezeichnet. Die Verseifung erfolgt in an sich bekannter Weise durch Zugabe einer Base, bevorzugt durch Zugabe einer Natrium- oder Kaliumhydroxidlösung in Wasser und/oder Alkohol. Besonders bevorzugt werden methanolische Natrium- oder Kaliumhydroxidlösungen eingesetzt, Die Verseifung wird bei Temperaturen im Bereich von 10 bis 80°C, bevorzugt im Bereich von 20 bis 60°C, durchgeführt. Der Verseifungsgrad hängt ab von der Menge der eingesetzten Base, von der Verseifungstemperatur, der Verseifungszeit und dem Wassergehalt der Lösung.

Der Verseifungsgrad der Polyvinylestergruppen liegt im Bereich von 1 bis 100 %, bevorzugt im Bereich von 40 bis 100 %, besonders bevorzugt im Bereich von 65 bis 100 %, ganz besonders bevorzugt im Bereich von 80 bis 100 %.

Die so hergestellten Polymerisate können durch Umsetzung von im Polymer vorhandenen Hydroxyl- und/oder Aminofunktionen mit Epoxiden der Formel VI nachträglich kationisiert werden (R²⁶ = C₁ bis C₄₀ Alkyl).

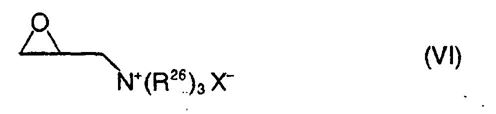

Dabei können bevorzugt die Hydroxylgruppen der Polyvinylalkohol-Einheiten und Vinylamin-Einheiten, entstanden durch Hydrolyse von Vinylformamid, mit den Epoxiden umgesetzt werden. Die Epoxide der Formel VI können auch in situ durch Umsetzung der entsprechenden Chlorhydrine mit Basen, beispielsweise Natriumhydroxid, erzeugt werden.
Bevorzugt wird 2,3-Epoxypropyl-trimethylammoniumchlorid bzw. 3-Chlor-2-hydroxypropyl-trimethylammoniumchlorid eingesetzt. Die K-Werte der Polymerisate sollen im Bereich von 10 bis 300, bevorzugt 25 bis 250, besonders bevorzugt 25 bis 200, ganz besonders bevorzugt im Bereich von 30 und 150, liegen. Der jeweils gewünschte K-Wert läßt sich in an sich bekannter Weise durch die Zusammensetzung der Einsatzstoffe einstellen. Die K-Werte werden bestimmt nach Fikentscher, Cellulosechemie, Bd. 13, S. 58 bis 64, und 71 bis 74 (1932) in N-Methylpyrrolidon bei 25°C und Polymerkonzentrationen, die je nach K-Wert-Bereich zwischen 0,1 Gew.-% und 5 Gew.-% liegen.

Nach der Verseifung können die Polymerlösungen zur Entfernung von Lösungsmitteln wasserdampfdestilliert werden. Nach der Wasserdampfdestillation erhält man je nach Verseifungsgrad, Art der Polyether b), der Vinylester a) und der eventuell eingesetzten Monomere c) wäßrige Lösungen oder Dispersionen.

Die erhaltenen Polymerisate können auch nachträglich vernetzt werden, indem man die Hydroxylgruppen bzw. Aminogruppen im Polymer mit mindestens bifunktionellen Reagentien umsetzt. Bei niedrigen Vernetzungsgraden erhält man wasserlösliche Produkte, bei hohen Vernetzungsgrade wasserquellbare bzw. unlösliche Produkte.

Beispielsweise können die erfindungsgemäßen Polymerisate mit Aldehyden, Dialdehyden, Ketonen und Diketonen, z.B. Formaldehyd, Acetaldehyd, Glyoxal, Glutaraldehyd, Succindialdehyd oder Terephthalaldehyd, umgesetzt werden. Desweiteren eignen sich aliphatische oder aromatische Carbonsäuren, beispielsweise Maleinsäure, Oxalsäure, Malonsäure, Succinsäure oder Citronensäure, bzw. Carbonsäurederivaten wie Carbonsäureester, -anhydride oder -halogenide. Ferner sind mehrfunktionelle Epoxide geeignet, z.B. Epichlorhydrin, Glycidylmethacrylat, Ethylenglykoldiglycidylether, 1,4-Butandioldiglycidylether oder 1,4-Bis(glycidyloxy)benzol. Ferner eigenen sich Diisocyanate, beispielsweise Hexamethylendiisocyanat, Isophorondiisocyanat, Methylendiphenyldiisocyanat, Toluylendiisocyanat oder Divinylsulfon.

Weiterhin eignen sich anorganische Verbindungen wie Borsäure oder Borsäuresalze, die im folgenden zusammenfassend als Borate bezeichnet werden, beispielsweise Natriummetaborat, Borax (Dinatriumtetraborat), sowie Salze mehrwertiger Kationen, z.B. Kupfer(II)salze wie Kupfer(II)acetat oder Zink-, Aluminium-, Titansalze.

Borsäure bzw. Borsäuresalze wie Natriummetaborat oder Dinatriumtetraborat eignen sich bevorzugt zur nachträglichen Vernetzung. Dabei können die Borsäure bzw. Borsäuresalze, bevorzugt als Salzlösungen, den Lösungen der erfindungsgemäßen Polymerisate zugegeben werden. Bevorzugt werden die Borsäure bzw. Borsäuresalze den wässerigen Polymerisatlösungen hinzugefügt.

Die Borsäure bzw. Borsäuresalze können den Polymerlösungen direkt nach der Herstellung zugefügt werden. Es ist aber auch möglich, die Borsäure bzw. Borsäuresalze nachträglich den kosmetischen Formulierungen mit den erfindungsgemäßen Polymerisaten zuzusetzen, bzw. während des Herstellungsprozesses der kosmetischen Formulierungen.

Der Anteil Borsäure bzw. Borsäuresalze bezogen auf die erfindungsgemäßen Polymere beträgt 0 bis 15 Gew-%, bevorzugt 0,1 bis 10 Gew.-%, besonders bevorzugt 0,5 bis 5 Gew.-%.

Die Polymerisatlösungen und -dispersionen können durch verschiedene Trocknungsverfahren wie z.B. Sprühtrocknung, Fluidized Spray Drying, Walzentrocknung oder Gefriertrocknung in Pulverform überführt werden. Als Trocknungsverfahren wird bevorzugt die Sprühtrocknung eingesetzt. Aus dem so erhaltenen Polymer-Trockenpulver läßt sich durch Lösen bzw. Redispergieren in Wasser erneut eine wäßrige Lösung bzw. Dispersion herstellen. Die Überführung in Pulverform hat den Vorteil einer besseren Lagerfähigkeit, einer einfacheren Transportmöglichkeit sowie eine geringere Neigung für Keimbefall.

Anstelle der wasserdampfdestillierten Polymerlösungen können auch die alkoholischen Polymerlösungen direkt in Pulverform überführt werden.

Die erfindungsgemäßen wasserlöslichen oder wasserdispergierbaren Polyalkylenoxid- bzw. Polyglycerin-haltigen Polymerisate eignen sich hervorragend zur Verwendung in haarkosmetischen Formulierungen.

Die erfindungsgemäßen Polymere, hergestellt durch radikalische Polymerisation von Vinylestern und optional weiterer polymerisierbarer Monomerer in Gegenwart polyetherhaltiger Verbindungen und anschließender zumindest teilweiser Verseifung der Esterfunktionen der ursprünglichen Vinylester, eignen sich als Stylingmittel und/oder Konditioniermittel in haarkosmetischen Zubereitungen wie Haarkuren, Haarlotionen, Haarspülungen, Haaremulsionen, Spitzenfluids, Egalisierungsmittel für Dauerwellen, "Hot-Oil-Treatment"-Präparate, Conditioner, Festigerlotionen oder Haarsprays. Je nach Anwendungsbegiet können die haarkosmetischen Zubereitungen als Spray, Schaum, Gel, Gelspray oder Mousse appliziert werden.

Die erfindungsgemäßen haarkosmetischen Formulierungen enthalten in einer bevorzugten Ausführungsform
a) 0,05 - 20 Gew.-% des erfindungsgemäßen Polymeren, hergestellt durch radikalische Polymerisation von Vinylestern und optional weiterer polymerisierbarer Monomerer in Gegenwart polyetherhaltiger Verbindungen und anschließender zumindest teilweiser Verseifung der Esterfunktionen der ursprünglichen Vinylester
b) 20 - 99,95 Gew.-% Wasser und/oder Alkohol
c) 0 - 79,5 Gew.-% weitere Bestandteile

Unter Alkohol sind alle in der Kosmetik üblichen Alkohole zu verstehen, z.B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d.h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z.B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Eiweißhydrolysate, Stabilisatoren, pH-Wert-Regulatoren, Farbstoffe, Viskositätsregulierer, Gelbildner, Farbstoffe, Salze, Feuchthaltemittel, Rückfetter und weitere übliche Additive.

Weiterhin zählen hierzu alle in der Kosmetik bekannten Stylingund Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise anionische Polymere. Solche anionischen Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane (Luviset® P.U.R.) und Polyharnstoffe. Besonders geeignete Polymere sind Copolymere aus t-Butylacrylat, Ethylacrylat, Methacrylsäure (z.B. Luvimer® 100P), Copolymere aus N-tert.-Butylacrylamid, Ethylacrylat, Acrylsäure (Ultrahold® 8, strong), Copolymere aus Vinylacetat, Crotonsäure und gegebenenfall weiteren Vinylestern (z.B. Luviset® Marken), Maleinsäureanhydridcopolymere, ggf. mit AIkoholen umgesetzt, anionische Polysiloxane, z.B. carboxyfunktionelle, Copolymere aus Vinylpyrrolidon, t-Butylacrylat, Methacrylsäure (z.B Luviskol® VBM).

Ganz besonders bevorzugt werden als anionische Polymere Acrylate mit einer Säurezahl größer gleich 120 und Copolymere aus t-Butylacrylat, Ethylacrylat, Methacrylsäure.

Weitere geeignete Haarkosmetik-Polymere sind kationische Polymere mit der Bezeichnung Polyquaternium nach INCI, z.B. Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam N-Vinylpyrrolidon/N-Vinylimidazoliumsalzen (Luviquat® Hold); kationische Cellulosederivate (Polyquaternium-4 und -10), Acrylamidcopolymere (Polyquaternium-7).

Als weitere Haarkosmetik-Polymere sind auch neutrale Polymere geeignet wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Polymere eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas). In einer bevorzugten Ausführungsform enthalten diese Zubereitungen
a) 0,1 - 10 Gew.-% des erfindungsgemäßen Polymeren, hergestellt durch radikalische Polymerisation von Vinylestern und optional weiterer polymerisierbarer Monomerer in Gegenwart polyetherhaltiger Verbindungen und anschließender zumindest teilweiser Verseifung der Esterfunktionen der ursprünglichen Vinylester
b) 20 - 99,9 Gew.-% Wasser und/oder Alkohol
c) 0 - 70 Gew.-% eines Treibmittel
d) 0 - 20 Gew.-% weitere Bestandteile

Treibmittel sind die für Haarsprays oder Aerosolschäume üblich verwendeten Treibmittel. Bevorzugt sind Gemische aus Propan/Butan, Pentan, Dimethylether, 1,1-Difluoreth (HFC-152 a), Kohlendioxid, Stickstoff oder Druckluft.

Eine erfindungsgemäß bevorzugte Formulierung für Aerosolhaarschäume enthält
a) 0,1 - 10 Gew.-% des erfindungsgemäßen Polymeren, hergestellt durch radikalische Polymerisation von Vinylestern und optional weiterer polymerisierbarer Monomerer in Gegenwart polyetherhaltiger Verbindungen und anschließender zumindest teilweiser Verseifung der Esterfunktionen der ursprünglichen Vinylester
b) 55 - 94,8 Gew.-% Wasser und/oder Alkohol
c) 5 - 20 Gew.-% eines Treibmittel
d) 0,1- 5 Gew.-% eines Emulgators
e) 0 - 10 Gew.-% weitere Bestandteile

Als Emulgatoren können alle in Haarschäumen üblicherwerse eingesetzen Emulgatoren verwendet werden. Geeignete Emulgatoren können nichtionisch, kationisch bzw. anionisch sein.
Beispiele für nichtionische Emulgatoren (INCI-Nomenklatur) sind Laurethe, z.B. Laureth-4; Cetethe, z.B. Cetheth-1, Polyethylenglycolcetylether; Cetearethe, z.B. Cetheareth-25, Polyglycolfettsäureglyceride, hydroxyliertes Lecithin, Lactylester von Fettsäuren, Alkylpolyglycoside.
Beispiele für kationische Emulgatoren sind Cetyldimethyl-2-hydroxyethylammoniumdihydrogenphosphat, Cetyltrimoniumchlorid, Cetyltrimmoniumbromid, Cocotrimoniummethylsulfat, Quaternium-1 bis x (INCI).
Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:
a) 0,1 - 10 Gew.-% des erfindungsgemäßen Polymeren, hergestellt durch radikalische Polymerisation von Vinylestern und optional weiterer polymerisierbarer Monomerer in Gegenwart polyetherhaltiger Verbindungen und anschließender zumindest teilweiser Verseifung der Esterfunktionen der ursprünglichen Vinylester
b) 60 - 99,85 Gew.-% Wasser und/oder Alkohol
c) 0,05 - 10 Gew.-% eines Gelbildners
d) 0 - 20 Gew.-% weitere Bestandteile

Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium 37 (and) Propylene Glycole Dicaprate Dicaprylate (and) PPG-1 Trideceth-6, Polyquaternium-7, Polyquaternium-44.

Die erfindungsgemäßen Polymere können auch in Shampooformulierungen als Festigungs- und/oder Konditioniermittel eingesetzt werden. Als Konditioniermittel eignen sich insbesondere Polymere mit kationischer Ladung. Bevorzugte Shampooformulierungen enthalten
a) 0,05 - 10 Gew.-% des erfindungsgemäßen Polymeren, hergestellt durch radikalische Polymerisation von Vinylestern und optional weiterer polymerisierbarer Monomerer in Gegenwart polyetherhaltiger Verbindungen und anschließender zumindest teilweiser Verseifung der Esterfunktionen der ursprünglichen Vinylester
b) 25 - 94,95 Gew.-% Wasser
c) 5 - 50 Gew.-% Tenside
c) 0 - 5 Gew.-% eines weiteren Konditioniermittels
d) 0 - 10 Gew.-% weitere kosmetische Bestandteile

In den Shampooformulierungen können alle in Shampoos üblicherweise eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsülfonat.

Geeignete amphotere Tenside sind zum Beispiel Alkylbetaine, Alkylamidopropylbetaine, Alkylsulfobetaine, Alkylglycinate, Alkylcarboxyglycinace, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt weden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z.B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den erfindungsgemäßen Polymeren eingesetzt werden. Hierzu zählen beispielsweise kationische Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA).

Ein weiterer Gegenstand der Erfindung sind Polymerisate, die erhältlich sind durch radikalische Polymerisation von
a) mindestens einem Vinylester einer C₁-C₂₄-Carbonsäure, in Gegenwart von
b) polyetherhaltigen Silikonderivaten und
c) gegebenenfalls eines oder mehrerer weiterer copolymerisierbarer Monomeren
und anschließender zumindest teilweiser Verseifung der Esterfunktionen der ursprünglichen Vinylester.

Bevorzugt sind Polymerisate, die erhältlich sind durch radikalische Polymerisation von
a) mindestens einem Vinylester einer C₁-C₂₄-Carbonnsäure, in Gegenwart von
b) polyetherhaltigen Silikonderivaten, die folgende Strukturelemente enthalten: 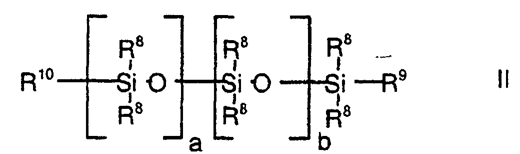 wobei:
   R⁹ = CH₃ oder 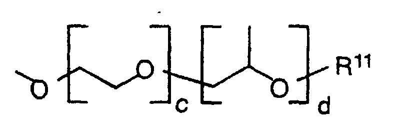
   R¹⁰ = CH₃ oder R⁹
   R¹¹ = H, CH₃, 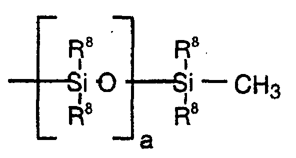 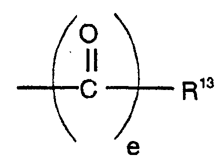
   R¹³ ein organischer Rest aus 1 bis 40 Kohlenstoffatomen, der Amino-, Carbonsäure- oder Sulfonatgruppen enthalten kann oder für den Fall e=0, auch das Anion einer anorganischen Säure bedeutet,
   und wobei die Reste R⁸ identisch oder unterschiedlich sein können, und entweder aus der Gruppe der aliphatischen Kohlenwasserstoffe mit 1 bis 20 Kohlenstoffatomen stammen, cyclische aliphatische Kohlenwasserstoffe mit 3 bis 20 C-Atomen sind, aromatischer Natur oder gleich R¹² sind, wobei:
   R¹² = 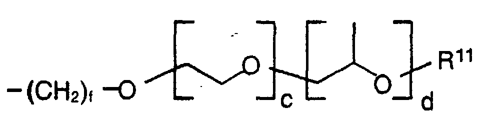
   mit der Maßgabe, daß mindestens einer der Reste R⁸, R⁹ oder R¹⁰ ein polyalkylenoxidhaltiger Rest nach obengenannter Definition ist,
   und f eine ganze Zahl von 1 bis 6 ist,
   a und b ganze Zahlen derart sind, daß das Molekulargewicht des Polysiloxan-Blocks zwischen 300 und 30000 liegt,
   c und d ganze Zahlen zwischen 0 und 50 sein können mit der Maßgabe, daß die Summe aus c und d größer als 0 ist, und e 0 oder 1 ist,
   und
c) gegebenenfalls eines oder mehreren weiteren copolymerisierbaren Monomeren
und anschließender zumindest teilweiser Verseifung der Vinylesterfunktionen der ursprünglichen Vinylester. Ganz besonders bevorzugt sind Polymerisate, die erhältlich sind durch radikalische Polymerisation von
a) mindestens einem Vinylester einer C₁-C₂₄-Carbonsäure, in Gegenwart von
b) polyetherhaltigen Silikonderivaten der allgemeinen Struktur: 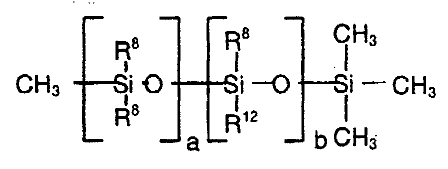 und
c) gegebenenfalls eines oder mehrerer weiterer copolymerisierbarer Monomeren
und anschließender zumindest teilweiser Verseifung der Esterfunktionen der ursprünglichen Vinylester a).

Ein weiterer Gegenstand der Erfindung sind Polymere, die erhältlich sind durch radikalische Polymerisation von
a) einem Vinylester einer C₁-C₂₄ Carbonsäure in Gegenwart von
b) polyetherhaltigen Verbindungen erhältlich durch Umsetzung von Polyethyleniminen mit Alkylenoxiden und
c) gegebenenfalls eines oder mehrerer weiterer copolymerisierbarer Monomeren
und anschließende zumindest teilweise Verseifung der Esterfunktionen der ursprünglichen Vinylester.

Ein weiterer Gegenstand der Erfindung sind Polymere, die erhältlich sind durch radikalische Polymerisation von
a) einem Vinylester einer C₁-C₂₄ Carbonsäure in Gegenwart von
b) Homo- und Copolymeren ethylenisch ungesättigten polyetherhaltigen Verbindungen und
c) gegebenenfalls eines oder mehrerer weiterer copolymerisierbarer Monomeren
und anschließende zumindest teilweise Verseifung der Esterfunktionen der ursprünglichen Vinylester.

Ein weiterer Gegenstand der Erfindung sind vernetzte Polymere, die erhältlich sind durch radikalische Polymerisation von
a) mindestens einem Vinylester von C₁-C₂₄-Carbonsäuren in Gegenwart von
b) polyetherhaltigen Verbindungen und
c) gegebenenfalls eines oder mehreren weiteren copolymerisierbaren Monomeren
und anschließender zumindest teilweiser Verseifung der Esterfunktionen der ursprünglichen Monomeren a), wobei der verwendete Vernetzer entweder bereits bei der Polymerisation zugegen ist oder im Anschluß an die Polymerisation und Verseifung zugegeben wird.

### Herstellungsbeispiele:

### Herstellvorschrift für Beispiele 1 bis 32

In einem Polymerisationsgefäß wird die polyetherhaltige Verbindung vorgelegt und unter Rühren und leichtem Stickstoffstrom auf 80°C erhitzt. Unter Rühren werden Vinylacetat und gegebenenfalls die weiteren Monomeren in 3 h zudosiert. Gleichzeitig wird eine Lösung von 1,4 g tert.-Butylperpivalat in 30 g Methanol ebenfalls in 3 h zugegeben. Danach wird noch 2 h bei 80°C gerührt. Nach dem Abkühlen wird das Polymerisat in 450 ml Methanol gelöst. Zur Verseifung gibt man bei 30°C 50 ml einer 10%igen methanolischen Natriumhydroxidlösung zu. Nach ca. 40 min. wird die Reaktion durch Zugabe von 750 ml 1%iger Essigsäure abgebrochen. Das Methanol wird durch Destillation entfernt.

Die K-Werte wurden 1%ig in N-Methylpyrrolidon bestimmt.

**Tabelle**

| Beispiel | Pfropfgrundlage | Vinylester | Comonomer | K-Wert | Verseifungsgrad[%] |
|---|---|---|---|---|---|
| 1 | PEG 1500¹ 72 g | Vinylacetat, 410 g | - | 47 | > 95 |
| 2 | PEG 4000 72 g | Vinylacetat, 410 g | - | 51 | > 95 |
| 3 | PEG 6000, 72 g | Vinylacetat, 410 g | - | 54 | > 95 |
| 4 | PEG 6000, 137 g | Vinylacetat, 410 g | | 49 | > 95 |
| 5 | PEG 6000, 22 g | Vinylacetat 410 g | - | 73 | > 95 |
| 6 | PEG 6000, 410 g | Vinylacetat 410 g | | 42 | > 95 |
| 7 | PEG 9000, 137 g | Vinylacetat, 410 g | - | 58 | > 95 |
| 8 | Polyglycerin 2200, 72 g | Vinylacetat, 410 g | - | 66 | > 95 |
| 9 | PEG-PPG-Blockcopolymer 8000², 72 g | Vinylacetat, 410 g | - | 45 | > 95 |
| 10 | Methylpolyethylenglykol 2000³ 72 g | Vinylacetat, 410 g | - | 47 | > 95 |
| 11 | Alkylpolyethylenglykol 3500⁴ 72 g | Vinylacetat, 410 g | - | 48 | > 95 |
| 12 | PPG 4000⁵ 72 | Vinylacetat 410 g | | 50 | > 95 |
| 13 | PEG 20000 72 g | Vinylacetat, 410 g | - | 69 | > 95 |
| 14 | PEG 20000 103 g | Vinylacetat, 410 g | - | 64 | > 95 |
| 15 | PEG 20000 137 g | Vinylacetat, 410 g | - | 59 | > 95 |
| 16 | PEG 20000 615 g | Vinylacetat, 410 g | - | 55 | 86 |
| 17 | PEG 35000 72 g | Vinylacetat, 410 g | - | 77 | > 95 |
| 18 | PEG 35000 137 g | Vinylacetat, 410 g | - | 80 | > 95 |
| 19 | PEG 35000 205 g | Vinylacetat, 410 g | - | 65 | 97 |
| 20 | Dimethicone copolyol⁶, 202 g | Vinylacetat, 410 g | - | 58 | > 95 |
| 21 | Poly(Natriummethacrylat-co-methylpolyethylenglykolmethacrylat)⁷ 103 g, | Vinylacetat, 410 g | | 43 | > 95 |
| 22 | ethoxyliertes Polyethylenimin⁸ | Vinylacetat, 410 g | | 52 | > 95 |
| 23 | PEG 6000, 72 g | Vinylacetat, 386 g | Methylmethacrylat, 24 g | 47 | > 95 |
| 24 | PEG 20000, 72 g | Vinylacetat, 328 g | N-Vinylpyrrolidon, 82 g | 61 | > 95 |
| 25 | PEG 20000, 72 g | Vinylacetat, 362 g | 3-Methyl-1-vinylimidazoliummethylsulfat, 48 g | 53 | > 95 |
| 26 | PEG 6000, 72 g | Vinylacetat, 367 g | N-Vinylformamid, 41 g | 57 | > 95 % |
| 27 | PEG 6000, 72 g | Vinylacetat, 326 g | N-Vinylformamid, 82 g | 67 | > 95 % |
| 28 | PEG 35000, 270 g | Vinylacetat, 410 g | | 59 | 96 |
| 29 | PEG 35000, 270 g | Vinylacetat, 410 g | Pentaerythrittriallyl-ether, 1,6 g | 71 | 95 |
| 30 | PEG 35000, 270 g | Vinylacetat, 410 g | Pentaerythrittriallyl-ether, 0,8 g | 65 | 94 |
| 31 | PEG 35000, 270 g | Vinylacetat, 410 g | N,N'-Divinyl-ethylenharnstoff 0,7 g | 73 | 95 |
| 32 | PEG 12000, 270 g | Vinylacetat, 410 g | Pentaerythrittriallyl-ether, 1,6 g | 50 | 94 |

| | | | | | |
|---|---|---|---|---|---|
| 1 PEG x: Polyethylenglykol mit mittlerem Molekulargewicht x (Gewichtsmittel) | | | | | |
| 2 Lutrol F 68 der Fa. BASF Aktiengesellschaft (PPG: Polypropylenglykol) | | | | | |
| 3 Pluriol A 2000 E der Fa. BASF Aktiengesellschaft | | | | | |
| 4 Lutensol AT 80 der Fa. BASF Aktiengesellschaft (C₁₆-C₁₈-Fettalkohol + 80 EO) | | | | | |
| 5 Polypropylenglykol mit mittlerem Molekulargewicht 4000 | | | | | |
| 6 Belsil DMC 6031TM der Fa. Wacker Chemie GmbH | | | | | |
| 7 Molverhältnis Natriummethacrylat/Methylpolyethylenglykolmethacrylat 4:1; Methylpolyethylenglykol mit Molmasse ca. 1000 | | | | | |
| 8 hergestellt aus 12,5 % Polyethylenimin (mittleres Molekulargewicht 1400) und 87,5 % Ethylenoxid | | | | | |

### Beispiel 33: Umsetzung mit 3-Chlor-2-hydroxypropyl-trimethylammoniumchlorid

Zu 400 g einer 32,9%igen Lösung aus Beispiel 3 gibt man 22 g einer 60%igen wässerigen Lösung von 3-Chlor-2-hydroxypropyltrimethylammoniumchlorid sowie 3,5 g Natriumhydroxid. Man rührt 3 Stunden bei 60°C und anschließend zwei weitere Stunden bei 90°C. Man erhält eine klare Lösung.

### Beispiel 34: Umsetzung mit 3-Chlor-2-hydroxypropyl-trimethylammoniumchlorid

Zu 400 g einer 15,3%igen Lösung aus Beispiel 26 gibt man 46 g einer 60%igen wässerigen Lösung von 3-Chlor-2-hydroxypropyltrimethylammoniumchlorid sowie 6 g Natriumhydroxid. Man rührt 3 Stunden bei 60°C und anschließend zwei weitere Stunden bei 90°C. Man erhält eine klare Lösung.

### Beispiel 35:

Man gibt bei Raumtemperatur unter Rühren innerhalb einer halben Stunde zu einer 19,3 %igen wässerigen Lösung des Polymers aus Beispiel 28 eine 5%ige wässerige Lösung von Dinatriumtetraboratdecahydrat (Borax). Man beobachtet einen Viskositätsanstieg.

| Menge an zugesetzter 5%iger Borax-Lösung [g] | Brookfieldviskosität (LVF, Spindel 2, 30 UpM, 23°C) [mPas] |
|---|---|
| 0 | 110 |
| 14,9 | 128 |
| 18,0 | 216 |
| 21,0 | 534 |
| 24,0 | 2228 |
| 26,9 | 7520¹ |
| 29,8 | 29190² |

| | |
|---|---|
| 1 Spindel 4, 30 UpM | |
| 2 Spindel 4, 6 UpM | |

### Formulierungsbeispiele:

### Beispiel 36:

| Formulierung Aerosolhaarschaum: |
|---|
| 2,00 % Copolymer aus Beispiel 3 |
| 2,00 % Luviquat Mono LS (Coco trimonium methyl sulfat) |
| 67,7 % Wasser |
| 10,0 Propan/Butan 3,5 bar (20°C) |
| q.s. Parfümöl |

### Beispiel 37 (Vergleichsbeispiel):

| |
|---|
| 2,00 % Polymergehalt Luviquat Hold (Polquaternium-46) |
| 2,00 % Luviquat Mono LS (Coco trimonium methyl sulfat) |
| 67,7 % Wasser |
| 10,0 Propan/Butan 3,5 bar (20°C) |
| q.s. Parfümöl |

Mit Beispiel 36 und Beispiel 37 (Vergleichbeispiel) wurden Halbseitentests an Modellköpfen durchgeführt. Die Beurteilung erfolgte subjektiv durch geschulte Friseure und Labormitarbeiter.

### Notenskala von 1 (sehr gut) bis 3 (schwach)

| | Beispiel 36 | Beispiel 37 (Vergleichsbeispiel) |
|---|---|---|
| Aufschäumen: | 1 | 1 |
| Konsistenz des Schaumes: | 1 | 1 |
| Verteilbarkeit: | 1 | 1 |
| Griff nasses Haar: | 1- | 2 |
| Naßkämmbarkeit: | 1- | 2+ |
| Festigung: | 1 | 2+ |
| Trockenkämmbarkeit: | 2+ | 2 |
| Klebrigkeit: | 1 | 1- |
| Griff des trockenenHaares: | 1- | 2+ |
| Elastizität des Haares | 1 | 2- |

Die Formulierung aus Beispiel 36 bewirkte im Vergleich zur Formulierung aus Beispiel 37 (Vergleichsbeispiel) eine bessere Festigung, eine bessere Naßkämmbarkeit, eine geringere Klebrigkeit sowie eine erhöhte Elastizität der Haare.

### Beispiel 38:

| Aerosolhaarschaum: | |
|---|---|
| | INCI |
| 4,00 % Copolymer aus Beispiel 19 | |
| 0,20 % Cremophor A 25 | Ceteareth-25 |
| 1,00 % Luviquat Mono CP | Hydroxyethyl cetyldimonium phosphate |
| 5, 00 % Ethanol | |
| 1,00 % Panthenol | |
| 10,0 Propan/Butan 3,5 bar (20°C) | |
| q.s. Parfümöl | |
| ad 100 % Wasser | |

### Beispiel 39:

| Pumpschaum: | |
|---|---|
| 2,00 % Copolymer aus Beispiel 7 | |
| 2,00 % Luviflex Soft (Polymergenalt) | |
| 1,20 % 2-Amino-2-methyl-1-propanol | |
| 0,20 % Cremophor A 25 | |
| 0,10 % Uvinul P 25 | PEG-25 PABA |
| q.s. Konservierungsmittel | |
| q.s. Parfümöl | |
| ad 100 % Wasser | |

### Beispiel 40:

| Pumpspray | |
|---|---|
| 4,00 % Copolymer aus Beispiel 17 | |
| 1,00 Panthenol | |
| 0,10 % Uvinul MS 40 | Benzophenone-4 |
| q.s. Konservierungsmittel | |
| q.s. Parfümöl | |
| ad 100 % Wasser | |

### Beispiel 41:

| Pumpspray: | |
|---|---|
| 4,00 % Copolymer aus Beispiel 9 | |
| 1,00 % Panthenol | |
| 0,10 % Uvinul M 40 | Benzophenone-3 |
| q.s. Konservierungsmittel | |
| q.s. Parfümöl | |
| ad 100 % Ethanol | |

### Beispiel 42:

| Haarspray: | |
|---|---|
| 5,00 % Copolymer aus Beispiel 6 | |
| 0,10 % Siliconöl Dow Corning DC 190 | Dimethicone Copolyol |
| 35,00 % Dimethylether | |
| 5,00 % n-Pentan | |
| ad 100 % Ethanol | |
| q.s. Parfümöl | |

### Beispiel 43:

| Haarspray VOC 55 %: | |
|---|---|
| 3,00 % Copolymer aus Beispiel 4 | |
| 7,00 % Luviset P.U.R. | Polyurethane-1 |
| 40,00 % Dimethylether | |
| 15,00 % Ethanol | |
| q.s. Parfümöl | |
| ad 100 % Wasser | |

### Beispiel 44:

| Haargel: | |
|---|---|
| 0,5 % Carbopol 980 | Cabomer |
| 3,00 % Copolymer aus Beispiel 18 | |
| 0,10 % Phythantriol | |
| 0,50 % Panthenol | |
| q.s. Parfümöl | |
| q.s Konservierungsmittel | |
| ad 100 % Wasser | |

### Beispiel 45:

| Haarshampoo bzw. Duschgel | |
|---|---|
| 0,5 % Copolymer aus Beispiel 33 | |
| 40,00 % Texapon NSO | Sodium Laureth Sulfate |
| 5,00 % Tego Betain L 7 | Cocamidopropyl Betaine |
| 5,00 % Plantacare 2000 | Decyl Glucoside |
| 1,00 % Propylenglycol | |
| q.s. Citronensäure | |
| q.s. Konservierungsmittel | |
| 1,00 % Natriumchlorid | |
| ad 100 % Wasser | |

## Patentansprüche

1. Verwendung von Polymerisaten, die erhältlich sind durch radikalische Polymerisation von
a) mindestens einem Vinylester von C₁-C₂₄-Carbonsäuren in Gegenwart von
b) polyetherhaltigen Verbindungen und
c) gegebenenfalls eines oder mehreren weiteren copolymerisierbaren Monomeren
und anschließender zumindest teilweiser Verseifung der Esterfunktionen der ursprünglichen Monomeren a), in haarkosmetischen Formulierungen.

2. Verwendung von Polymerisaten nach Anspruch 1, **dadurch gekennzeichnet, daß** die Polymerisate erhältlich sind durch radikalische Polymerisation von
a) mindestens einem Vinylester von C₁-C₂₄-Carbonsäuren in Gegenwart von
b) polyetherhaltigen Verbindungen der allgemeinen Formel I 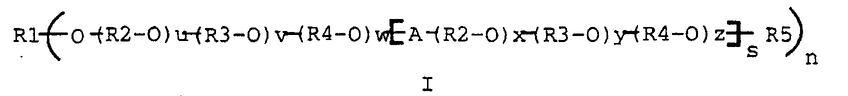 in der die Variablen unabhängig voneinander folgende Bedeutung haben:
R¹ Wasserstoff, C₁-C₂₄-Alkyl, R⁶-C(=O)-, R⁶-NH-C(=O)-, Polyalkoholrest;
R⁵ Wasserstoff, C₁-C₂₄-Alkyl, R⁶-C(=O)-, R⁶-NH-C(=O)-;
R² bis R⁴ -(CH₂)₂-, -(CH₂)₃-, -(CH₂)₄-, -CH₂-CH(R⁶)-,
-CH₂-CHOR⁷-CH₂-;
R⁶ C₁-C₂₄-Alkyl;
R⁷ Wasserstoff, C₁-C₂₄-Alkyl, R⁶-C(=O)-, R⁶-NH-C(=O)-;
A -C(=O)-O, -C(=O)-B-C(=O)-O,
-C(=O)-NH-B-NH-C(=O)-O;
B -(CH₂)ₜ-, Arylen, ggf. substituiert;
n 1 bis 1000;
s 0 bis 1000;
t 1 bis 12;
u 1 bis 5000;
v 0 bis 5000;
w 0 bis 5000;
x 0 bis 5000;
y 0 bis 5000;
z 0 bis 5000;
und
c) gegebenenfalls eines oder mehreren weiteren copolymerisierbaren Monomeren
und anschließender zumindest teilweiser Verseifung der Esterfunktionen der ursprünglichen Monomeren a).

3. Verwendung von Polymerisaten nach Anspruch 2, **dadurch gekennzeichnet, daß** die Polymerisate erhältlich sind durch radikalische Polymerisation von
a) mindestens einem Vinylester von C₁-C₂₄-Carbonsäuren in Gegenwart von
b) polyetherhaltigen Verbindungen der allgemeinen Formel I mit einem mittleren Molekulargewicht von 300 bis 100000 (nach dem Zahlenmittel), in der die Variablen unabhängig voneinander folgende Bedeutung haben:
R¹ Wasserstoff, C₁-C₁₂-Alkyl, R⁶-C(=O)-, R⁶-NH-C(=O)-, Polyalkoholrest;
R⁵ Wasserstoff, C₁-C₁₂-Alkyl, R⁶-C(=O)-, R⁶-NH-C(=O)-;
R² bis R⁴ -(CH₂)₂-, -(CH₂)₃-, -(CH₂)₄-, -CH₂-CH(R⁶)-,
-CH₂-CHOR⁷-CH₂-;
R⁶ C₁-C₁₂-Alkyl;
R⁷ Wasserstoff, C₁-C₁₂-Alkyl, R⁶-C(=O)-, R⁶-NH-C(=O)-;
n 1 bis 8;
s 0;
u 2 bis 2000;
v 0 bis 2000;
w 0 bis 2000;
und
c) gegebenenfalls eines oder mehreren weiteren copolymerisierbaren Monomeren
und anschließender zumindest teilweiser Verseifung der Esterfunktionen der ursprünglichen Monomeren a).

4. Verwendung von Polymerisaten nach Anspruch 2, **dadurch gekennzeichnet, daß** die Polymerisate erhältlich sind durch radikalische Polymerisation von
a) mindestens einem Vinylester von C₁-C₂₄-Carbonsäuren in Gegenwart von
b) polyetherhaltigen Verbindungen der allgemeinen Formel I mit einem mittleren Molekulargewicht von 500 bis 50000 (nach dem Zahlenmittel), in der die Variablen unabhängig voneinander folgende Bedeutung haben:
R¹ Wasserstoff, C₁-C₆-Alkyl, R⁶-C(=O)-, R⁶-NH-C(=O)-;
R⁵ Wasserstoff, C₁-C₆-Alkyl, R⁶-C(=O)-, R⁶-NH-C(=O)-;
R² bis R⁴ -(CH₂)₂-, -(CH₂)₃-, -(CH₂)₄-, -CH₂-CH(R⁶)-,
-CH₂-CHOR⁷-CH₂-;
R⁶ C₁-C₆-Alkyl;
R⁷ Wasserstoff, C₁-C₆-Alkyl, R⁶-C(=O)-, R⁶-NH-C(=O)-;
n 1;
s 0;
u 5 bis 500;
v 0 bis 500;
w 0 bis 500;
und
c) gegebenenfalls eines oder mehreren weiteren copolymerisierbaren Monomeren
und anschließender zumindest teilweiser Verseifung der Esterfunktionen der ursprünglichen Monomeren a), in haarkosmetischen Formulierungen.

5. Verwendung von Polymerisaten nach Anspruch 1, **dadurch gekennzeichnet, daß** die Polymerisate erhältlich sind durch radikalische Polymerisation von
a) mindestens einem Vinylester von C₁-C₂₄-Carbonsäuren in Gegenwart von
b) polyetherhaltigen Silikonderivaten
und
c) gegebenenfalls eines oder mehrerer weiterer copolymerisierbarer Monomeren
und anschließender zumindest teilweiser Verseifung der Esterfunktion der ursprünglichen Monomeren a).

6. Verwendung von Polymerisaten nach Anspruch 5, **dadurch gekennzeichnet, daß** die Polymerisate erhältlich sind durch radikalische Polymerisation von
a) mindestens einem Vinylester von C₁-C₂₄-Carbonsäuren in Gegenwart von
b) polyetherhaltigen Silikonderivaten der allgemeinen Formel II 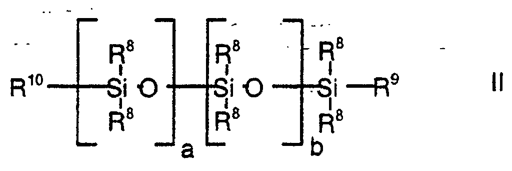
wobei:
R⁹ = CH₃ oder 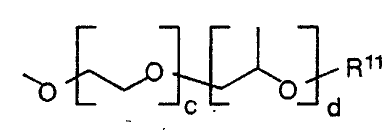
R¹⁰ = CH₃ oder R⁹
R¹¹ = H, CH₃, 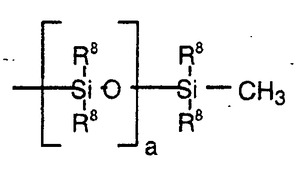 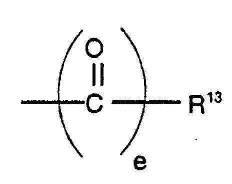
R¹³ ein organischer Rest aus 1 bis 40 Kohlenstoffatomen, der Amino-, Carbonsäure- oder Sulfonatgruppen enthalten kann, oder für den Fall e=0, auch das Anion einer anorganischen Säure bedeutet,
und wobei die Reste R⁸ identisch oder unterschiedlich sein können, und entweder aus der Gruppe der aliphatischen Kohlenwasserstoffe mit 1 bis 20 Kohlenstoffatomen stammen, cyclische aliphatische Kohlenwasserstoffe mit 3 bis 20 C-Atomen sind, aromatischer Natur oder gleich R¹² sind, wobei:
R¹² = 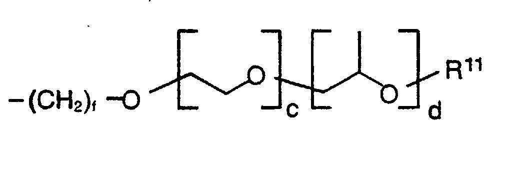
mit der Maßgabe, daß mindestens einer der Reste R⁸, R⁹ oder R¹⁰ ein polyalkylenoxidhaltiger Rest nach obengenannter Definition, ist,
und f eine ganze Zahl von 1 bis 6 ist,
a und b ganze Zahlen derart sind, daß das Molekulargewicht des Polysiloxan-Blocks zwischen 300 und 30000 liegt, c und d ganze Zahlen zwischen 0 und 50 sein können mit der Maßgabe, daß die Summe aus c und d größer als 0 ist, und e 0 oder 1 ist,
und
gegebenenfalls eines oder mehrerer weiterer copolymerisierbarer Monomeren und anschließender zumindest teilweiser Verseifung der Esterfunktionen der ursprünglichen Monomeren a).

7. Verwendung von Polymerisaten nach Anspruch 6, **dadurch gekennzeichnet, daß** Formel II folgende Bedeutung besitzt:

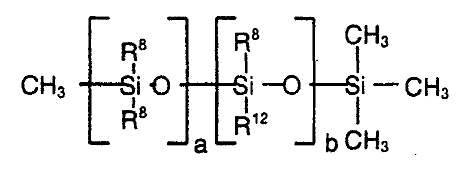

8. Verwendung nach Anspruch 1, **dadurch gekennzeichnet, daß** die Polymerisate erhältlich sind durch radikalische Polymerisation von
a) mindestens einem Vinylester von C₁-C₂₄-Carbonsäuren in Gegenwart von
b) polyetherhaltigen Verbindungen erhältlich durch Umsetzung von Polyethyleniminen mit Alkylenoxiden
und
c) gegebenenfalls eines oder mehrerer weiterer copolymerisierbarer Monomeren
und anschließender zumindest teilweiser Verseifung der Esterfunktionen der ursprünglichen Monomeren a).

9. Verwendung von Polymerisaten nach Anspruch 8, **dadurch gekennzeichnet, daß** als Alkylenoxide Ethylenoxid, Propylenoxid, Butylenoxid und Mischungen aus diesen verwendet werden.

10. Verwendung von Polymerisaten nach Ansprüchen 8 und 9, **dadurch gekennzeichnet, daß** als Alkylenoxid Ethylenoxid verwendet wird.

11. Verwendung von Polymerisaten nach Ansprüchen 8, 9 und 10, **dadurch gekennzeichnet, daß** das Polyethylenimin ein Molekulargewicht zwischen 300 und 20000 bsitzt.

12. Verwendung von Polymerisaten nach Anspruch 1, **dadurch gekennzeichnet, daß** die polyetherhaltigen Verbindungen b) durch Polymerisation von ethylenisch ungesättigten alkylenoxidhaltigen Monomeren und gegebenenfalls weiteren copolymerisierbaren Monomeren hergestellt worden sind.

13. Verwendung von Polymerisaten nach Anspruch 12, **dadurch gekennzeichnet, daß** die polyetherhaltigen Verbindungen b) durch Polymerisation von Polyalkylenoxidvinylethern und gegebenenfalls weiteren copolymerisierbaren Monomeren hergestellt worden sind.

14. Verwendung von Polymerisaten nach Anspruch 12, **dadurch gekennzeichnet, daß** die polyetherhaltigen Verbindungen b) durch Polymerisation von Polyalkylenoxid(meth)acrylaten und gegebenenfalls weiteren copolymerisierbaren Monomeren hergestellt worden sind.

15. Verwendung von Polymerisaten nach Ansprüchen 1 bis 14, **dadurch gekennzeichnet, daß** c) ausgewählt wird aus der Gruppe:
Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, Stearylacrylat, Stearylmethacrylat, N-t-Butylacrylamid, N-Octylacrylamid, 2-Hydroxyethylacrylat, Hydroxypropylacrylate, 2-Hydroxyethylmethacrylat, Hydroxypropylmethacrylate, Alkylenglykol(meth)acrylate, Styrol, ungesättigte Sulfonsäuren wie z.B. Acrylamidopropansulfonsäure, Vinylpyrrolidon, Vinylcaprolactam, Vinylether (z.B.: Methyl-, Ethyl-, Butyl- oder Dodecylvinylether), Vinylformamid, Vinylmethylacetamid, Vinylamin, 1-Vinylimidazol, 1-Vinyl-2-methylimidazol, N,N-Dimethylaminomethylmethacrylat und
N-[3-(dimethylamino)propyl]methacrylamid; 3-Methyl-1-vinylimidazoliumchlorid, 3-Methyl-1-vinylimidazoliummethylsulfat, N,N-Dimethylaminoethylmethacrylat, N-[3-(dimethylamino)-propyl]methacrylamid quaternisiert mit Methylchlorid, Methylsulfat oder Diethylsulfat.

16. Verwendung von Polymerisaten nach Ansprüchen 1 und 15, **dadurch gekennzeichnet, daß** die Mengenverhältnisse
a) 10 - 90 Gew.-%
b) 2 - 90 Gew.-%
c) 0 - 50 Gew.-%
betragen.

17. Verwendung von Polymerisaten nach Ansprüchen 1 bis 15, **dadurch gekennzeichnet, daß** die Mengenverhältnisse
a) 50 - 97 Gew.-%
b) 3 - 50 Gew.-%
c) 0 - 30 Gew.-%
betragen.

18. Verwendung von Polymerisaten nach Ansprüchen 1 bis 15, **dadurch gekennzeichnet, daß** die Mengenverhältnisse
a) 60 - 97 Gew.-%
b) 3 - 40 Gew.-%
c) 0 - 20 Gew.-%
betragen.

19. Verwendung nach Anspruch 1 bis 18, wobei im Anschluß an die Verseifung eine Vernetzung durchgeführt wird.

20. Verwendung nach Anspruch 19, wobei die Vernetzung durch Aldehyde, Dialdehyde oder Borate erfolgt.

21. Haarkosmetische Formulierungen, **dadurch gekennzeichnet, daß** sie wie folgt zusammengesetzt sind:
a) 0,05 - 20 Gew.-% des Polymeren gemäß Anspruch 1
b) 20 - 99,95 Gew.-% Wasser und/oder Alkohol
c) 0 - 79,05 Gew.-% weitere Bestandteile

22. Haarkosmetische Formulierungen, **dadurch gekennzeichnet, daß** sie wie folgt zusammengesetzt sind:
a) 0,1 - 10 Gew.-% des Polymeren gemäß Anspruch 1
b) 20 - 99,9 Gew.-% Wasser und/oder Alkohol
c) 0 - 70 Gew.-% eines Treibmittel
d) 0 - 20 Gew.-% weiterer Bestandteile

23. Haarkosmetische Formulierungen, **dadurch gekennzeichnet, daß** sie wie folgt zusammengesetzt sind:
a) 0,1 - 10 Gew.-% des Polymeren gemäß Anspruch 1
b) 55 - 94,8 Gew.-% Wasser und/oder Alkohol
c) 5 - 20 Gew.-% eines Treibmittel
d) 0,1 - 5 Gew.-% eines Emulgators
e) 0 - 10 Gew.-% weiterer Bestandteile

24. Haarkosmetische Formulierungen, **dadurch gekennzeichnet, daß** sie wie folgt zusammengesetzt ist:
a) 0,1 - 10 Gew.-% des Polymeren gemäß Anspruch 1
b) 60 - 99,85 Gew.-% Wasser und/oder Alkohol
c) 0,05 - 10 Gew.-% eines Gelbildners
d) 0 - 20 Gew.-% weitere Bestandteile

25. Haarkosmetische Formulierungen, **dadurch gekennzeichnet, daß** sie wie folgt zusammengesetzt ist:
a) 0,05 - 10 Gew.-% des Polymeren gemäß Anspruch 1,
b) 25 - 94,95 Gew.-% Wasser
c) 5 - 50 Gew.-% Tenside
d) 0 - 5 Gew.-% eines weiteren Konditioniermittels
e) 0 - 10 Gew.-% weiterer kosmetische Bestandteile

26. Polymerisate, die erhältlich sind durch radikalische Polymerisation von
a) mindestens einem Vinylester einer C₁-C₂₄-Carbonsäure, in Gegenwart von
b) polyetherhaltigen Silikonderivaten und
c) gegebenenfalls eines oder mehrerer-weiterer copolymerisierbarer Monomeren
und anschließender zumindest teilweiser Verseifung der Esterfunktionen der ursprünglichen Monomere a).

27. Polymere, die erhältlich sind durch radikalische Polymerisation von
a) einem Vinylester einer C₁-C₂₄-Carbonsäure in Gegenwart von
b) polyetherhaltigen Verbindungen erhältlich durch Umsetzung von Polyethyleniminen mit Alkylenoxiden und
c) gegebenenfalls eines oder mehrerer weiterer copolymerisierbarer Monomeren
und anschließender zumindest teilweiser Verseifung der Esterfunktionen der ursprünglichen Monomere a).

28. Polymere, die erhältlich sind durch radikalische Polymerisation von
a) einem Vinylester einer C₁-C₂₄-Carbonsäure in Gegenwart von
b) Homo- und Copolymeren ethylenisch ungesättigten polyetherhaltigen Verbindungen und
c) gegebenenfalls eines oder mehrerer weiterer copolymerisierbarer Monomeren
und anschließende zumindest teilweise Verseifung der Esterfunktionen der ursprünglichen Monomere a).

29. Vernetzte Polymere, die erhältlich sind durch radikalische Polymerisation von
a) mindestens einem Vinylester von C₁-C₂₄-Carbonsäuren in Gegenwart von
b) polyetherhaltigen Verbindungen und
c) gegebenenfalls eines oder mehreren weiteren copolymerisierbaren Monomeren
und anschließender zumindest teilweiser Verseifung der Esterfunktionen der ursprünglichen Monomeren a), wobei der verwendete Vernetzer entweder bereits bei der Polymerisation zugegen ist oder im Anschluß an die Polymerisation und Verseifung zugegeben wird.

30. Vernetzte Polymere nach Anspruch 29, wobei als Vernetzer Aldehyde, Dialdehyde oder Borate verwendet werden.

31. Vernetzte Polymere nach Anspruch 29, **dadurch gekennzeichnet, daß** der Vernetzer bereits bei der Polymerisation zugegen ist.

## Claims

1. The use of polymers obtainable by free-radical polymerization of
a) at least one vinyl ester of C₁-C₂₄-carboxylic acids in the presence of
b) polyether-containing compounds and
c) optionally one or more other copolymerizable monomers
and subsequent at least partial hydrolysis of the ester functions of the original monomers a), in hair cosmetic formulations.

2. The use of polymers as claimed in claim 1, wherein the polymers are obtainable by free-radical polymerization of
a) at least one vinyl ester of C₁-C₂₄-carboxylic acids in the presence of
b) polyether-containing compounds of the formula I 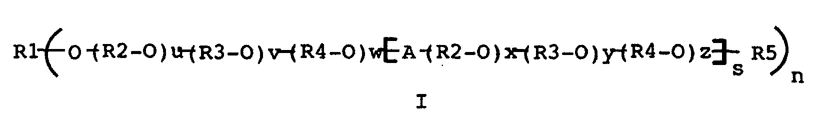 in which the variables independently of one another have the following meanings:
R¹ is hydrogen, C₁-C₂₄-alkyl, R⁶-C(=O)-, R⁶-NH-C(=O)-, polyalcohol radical;
R⁵ is hydrogen, C₁-C₂₄-alkyl, R⁶-C(=O)-, R⁶-NH-C(=O)-;
R² to R⁴ are
-(CH₂)₂-, -(CH₂)₃-, -(CH₂)₄-, -CH₂-CH(R⁶)-,
-CH₂-CHOR⁷-CH₂-;
R⁶ is C₁-C₂₄-alkyl;
R⁷ is hydrogen, C₁-C₂₄-alkyl, R⁶-C(=O)-, R⁶-NH-C(=O)-;
A is -C(=O)-O, -C(=O)-B-C(=O)-O,
-C(=O)-NH-B-NH-C(=O)-O;
B is -(CH₂)ₜ-, arylene, optionally substituted;
n is from 1 to 1000;
s is from 0 to 1000;
t is from 1 to 12;
u is from 1 to 5000;
v is from 0 to 5000;
w is from 0 to 5000;
x is from 0 to 5000;
y is from 0 to 5000;
z is from 0 to 5000;
and
c) optionally one or more other copolymerizable monomers
and subsequent at least partial hydrolysis of the ester functions of the original monomers a).

3. The use of polymers as claimed in claim 2, wherein the polymers are obtainable by free-radical polymerization of
a) at least one vinyl ester of C₁-C₂₄-carboxylic acids in the presence of
b) polyether-containing compounds of the formula I having an average molecular weight of from 300 to 100000 (number average), in which the variables independently of one another have the following meanings:
R¹ is hydrogen, C₁-C₁₂-alkyl, R⁶-C(=O)-, R⁶-NH-C(=O)-, polyalcohol radical;
R⁵ is hydrogen, C₁-C₁₂-alkyl, R⁶-C(=O)-, R⁶-NH-C(=O)-;
R² to R⁴ are
-(CH₂)₂-, -(CH₂)₃-, -(CH₂)₄-, -CH₂-CH(R⁶)-,
-CH₂-CHOR⁷-CH₂-;
R⁶ is C₁-C₁₂-alkyl;
R⁷ is hydrogen, C₁-C₁₂-alkyl, R⁶-C(=O)-, R⁶-NH-C(=O)-;
n is from 1 to 8;
s is 0;
u is from 2 to 2000;
v is from 0 to 2000;
w is from 0 to 2000;
and
c) optionally one or more other copolymerizable monomers
and subsequent at least partial hydrolysis of the ester functions of the original monomers a).

4. The use of polymers as claimed in claim 2, wherein the polymers are obtainable by free-radical polymerizable of
a) at least one vinyl ester of C₁-C₂₄-carboxylic acids in the presence of
b) polyether-containing compounds of the formula I having an average molecular weight of from 500 to 50000 (number average), in which the variables independently of one another have the following meaning:
R¹ is hydrogen, C₁-C₆-alkyl, R⁶-C(=O)-, R⁶-NH-C(=O)-;
R⁵ is hydrogen, C₁-C₆-alkyl, R⁶-C(=O)-, R⁶-NH-C(=O)-;
R² to R⁴ are
-(CH₂)₂-, -(CH₂)₃-, -(CH₂)₄-, -CH₂-CH(R⁶)-,
-CH₂-CHOR⁷-CH₂-;
R⁶ is C₁-C₆-alkyl;
R⁷ is hydrogen, C₁-C₆-alkyl, R⁶-C(=O)-, R⁶-NH-C(=O)-;
n is 1;
s is 0;
u is from 5 to 500;
v is from 0 to 500;
w is from 0 to 500;
and
c) optionally at least one or more other copolymerizable monomers
and subsequent at least partial hydrolysis of the ester functions of the original monomers a), in hair cosmetic formulations.

5. The use of polymers as claimed in claim 1, wherein the polymers are obtainable by free-radical polymerization of
a) at least one vinyl ester of C₁-C₂₄-carboxylic acids in the presence of
b) polyether-containing silicone derivatives
and
c) optionally one or more other copolymerizable monomers
and subsequent at least partial hydrolysis of the ester function of the original monomers a).

6. The use of polymers as claimed in claim 5, wherein the polymers are obtainable by free-radical polymerisation of
a) at least one vinyl ester of C₁-C₂₄-carboxylic acids in the presence of
b) polyether-containing silicone derivatives of the formula II 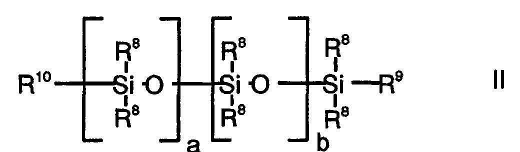
where:
R⁹ = CH₃ or 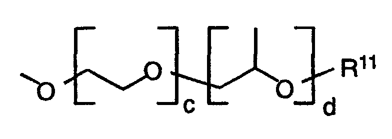
R¹⁰ = CH₃ or R⁹
R¹¹ = H, CH₃, 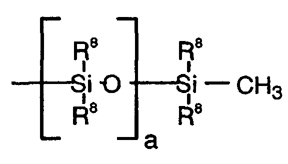 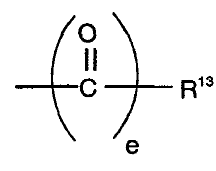
R¹³ is a C₁-C₄₀ organic radical which can contain amino, carboxyl or sulfonate groups, or where e = 0, is also the anion of an inorganic acid,
and where the radicals R⁸ can be identical or different, and come either from the group of aliphatic hydrocarbons having from 1 to 20 carbon atoms, are cyclic aliphatic hydrocarbons having from 3 to 20 carbon atoms, are of an aromatic nature or are identical to R¹², where:
R¹² = 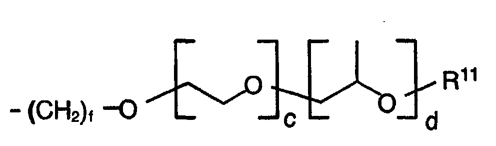
with the proviso that at least one of the radicals R⁸, R⁹ or R¹⁰ is a polyalkylene oxide-containing radical as defined above,
and f is an integer from 1 to 6,
a and b are integers such that the molecular weight of the polysiloxane block is between 300 and 30000,
c and d can be integers between 0 and 50, with the proviso that the sum c + d is greater than 0, and e is 0 or 1,
and
optionally one or more other copolymerizable monomers and subsequent at least partial hydrolysis of the ester functions of the original monomers a).

7. The use of polymers as claimed in claim 6, wherein formula II has the following meaning:

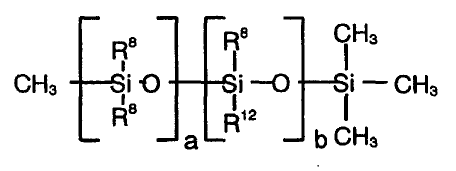

8. The use as claimed in claim 1, wherein the polymers are obtainable by free-radical polymerization of
a) at least one vinyl ester of C₁-C₂₄-carboxylic acids in the presence of
b) polyether-containing compounds obtainable by reaction of polyethyleneimines with alkylene oxides
and
c) optionally one or more other copolymerizable monomers
and subsequent at least partial hydrolysis of the ester functions of the original monomers a).

9. The use of polymers as claimed in claim 8, wherein the alkylene oxides used are ethylene oxide, propylene oxide, butylene oxide and mixtures thereof.

10. The use of polymers as claimed in claims 8 and 9, wherein the alkylene oxide used is ethylene oxide.

11. The use of polymers as claimed in claims 8, 9 and 10, wherein the polyethyleneimine has a molecular weight between 300 and 20000.

12. The use of polymers as claimed in claim 1, wherein the polyether-containing compounds b) have been prepared by polymerization of ethylenically unsaturated alkylene oxide-containing monomers and optionally other copolmerizable monomers.

13. The use of polymers as claimed in claim 12, wherein the polyether-containing compounds b) have been prepared by polymerization of polyalkylene oxide vinyl ethers and optionally other copolymerizable monomers.

14. The use of polymers as claimed in claim 12, wherein the polyether-containing compounds b) have been prepared by polymerization of polyalkylene oxide (meth)acrylates and optionally other copolymerizable monomers.

15. The use of polymers as claimed in claims 1 to 14, wherein c) is chosen from the group:
acrylic acid, methacrylic acid, maleic acid, fumaric acid, crotonic acid, maleic anhydride and its half-esters, methyl acrylate, methyl methacrylate, ethyl acrylate, ethyl methacrylate, n-butyl acrylate, n-butyl methacrylate, t-butyl acrylate, t-butyl methacrylate, isobutyl acrylate, isobutyl methacrylate, 2-ethylhexyl acrylate, stearyl acrylate, stearyl methacrylate, N-t-butylacrylamide, N-octylacrylamide, 2-hydroxyethyl acrylate, hydroxypropyl acrylates, 2-hydroxyethyl methacrylate, hydroxypropyl methacrylates, alkylene glycol (meth)acrylates, styrene, unsaturated sulfonic acids such as, for example, acrylamidopropane sulfonic acid, vinyl pyrrolidone, vinyl caprolactam, vinyl ethers, (e.g. methyl, ethyl, butyl or dodecyl vinyl ethers), vinylformamide, vinylmethylacetamide, vinylamine, 1-vinylimidazole, 1-vinyl-2-methylimidazole, N,N-dimethylaminomethyl methacrylate and N-[3-(dimethylamino)propyl]methacrylamide; 3-methyl-1-vinylimidazolium chloride, 3-methyl-1-vinylimidazolium methylsulfate, N,N-dimethylaminoethyl methacrylate, N-[3-(dimethylamino)propyl]methacrylamide quaternized with methyl chloride, methyl sulfate or diethyl sulfate.

16. The use of polymers as claimed in claims 1 and 15, wherein the quantitative ratios are
a) 10 - 90 % by weight
b) 2 - 90 % by weight
c) 0 - 50 % by weight.

17. The use of polymers as claimed in claims 1 to 15, wherein the quantitative ratios are
a) 50 - 97 % by weight
b) 3 - 50 % by weight
c) 0 - 30 % by weight.

18. The use of polymers as claimed in claims 1 to 15, wherein the quantitative ratios are
a) 60 - 97 % by weight
b) 3 - 40 % by weight
c) 0 - 20 % by weight.

19. The use as claimed in claims 1 to 18, where a crosslinking is carried out after the hydrolysis.

20. The use as claimed in claim 19, where the crosslinking is carried out by aldehydes, dialdehydes or borates.

21. A hair cosmetic formulation which has the following composition:
a) 0.05 - 20 % by weight of the polymer as in claim 1
b) 20 - 99.95 % by weight of water and/or alcohol
c) 0 - 79.05 % by weight of other constituents.

22. A hair cosmetic formulation which has the following composition:
a) 0.1 - 10 % by weight of the polymer as in claim 1
b) 20 - 99.9 % by weight of water and/or alcohol
c) 0 - 70 % by weight of a propellant
d) 0 - 20 % by weight of other constituents.

23. A hair cosmetic formulation which has the following composition:
a) 0.1 - 10 % by weight of the polymer as in claim 1
b) 55 - 94.8 % by weight of water and/or alcohol
c) 5 - 20 % by weight of a propellant
d) 0.1 - 5 % by weight of an emulsifier
e) 0 - 10 % by weight of other constituents.

24. A hair cosmetic formulation which has the following composition:
a) 0.1 - 10 % by weight of the polymer as in claim 1
b) 60- 99.85 % by weight of water and/or alcohol
c) 0.05 - 10 % by weight of a gel former
d) 0 - 20 % by weight of other constituents.

25. A hair cosmetic formulation which has the following composition:
a) 0.05 - 10 % by weight of the polymer as in claim 1,
b) 25 - 94.95 % by weight of water
c) 5 - 50 % by weight of surfactants
d) 0 - 5 % by weight of another conditioning agent
e) 0 - 10 % by weight of other cosmetic constituents.

26. A polymer obtainable by free-radical polymerization of
a) at least one vinyl ester of a C₁-C₂₄ carboxylic acid, in the presence of
b) polyether-containing silicone derivatives and
c) optionally one or more other copolymerizable monomers
and subsequent at least partial hydrolysis of the ester functions of the original monomers a).

27. A polymer obtainable by free-radical polymerization of
a) a vinyl ester of a C₁-C₂₄ carboxylic acid in the presence of
b) polyether-containing compounds obtainable by reaction of polyethyleneimines with alkylene oxides and
c) optionally one or more other copolymerizable monomers
and subsequent at least partial hydrolysis of the ester functions of the original monomers a).

28. A polymer obtainable by free-radical polymerization of
a) a vinyl ester of a C₁-C₂₄ carboxylic acid in the presence of
b) homo- and copolymers of ethylenically unsaturated polyether-containing compounds and
c) optionally one or more other copolymerizable monomers
and subsequent at least partial hydrolysis of the ester functions of the original monomers a).

29. A crosslinked polymer obtainable by free-radical polymerization of
a) at least one vinyl ester of C₁-C₂₄ carboxylic acids in the presence of
b) polyether-containing compounds and
c) optionally one or more further copolymerizable monomers
and subsequent at least partial hydrolysis of the ester functions of the original monomers a), where the crosslinker used is either already present during the polymerization, or is added after the polymerization and hydrolysis.

30. A crosslinked polymer as claimed in claim 29, where the crosslinkers used are aldehydes, dialdehydes or borates.

31. The crosslinked polymer as claimed in claim 29, wherein the crosslinker is already present during the polymerization.

## Revendications

1. Utilisation de polymères obtenus par polymérisation radicalaire de
a) au moins un ester vinylique d'acide carboxylique en C1-C24 en présence de
b) des composés contenant des polyéthers et
c) le cas échéant un ou plusieurs autres monomères copolymérisables,
suivie d'une saponification au moins partielle des fonctions ester des monomères initiaux a), dans des compositions cosmétiques capillaires.

2. Utilisation de polymères selon la revendication 1, **caractérisé par le fait qu'**ils sont obtenus par polymérisation radicalaire de
a) au moins un ester vinylique d'acide carboxylique en C1-C24 en présence de
b) des composés contenant des polyéthers et répondant à la formule générale I 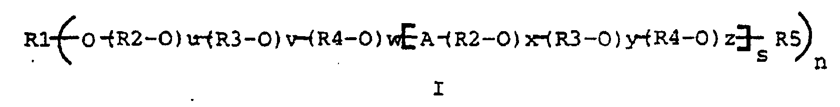 dans laquelle les symboles ont, indépendamment les uns des autres, les significations suivantes :
R¹ : l'hydrogène, un groupe alkyle en C1-C24, R⁶-C(=O)-, R⁶-NH-C(=O)-, un radical de polyalcool ;
R⁵ : l'hydrogène, un groupe alkyle en C1-C24, R⁶-C(=O)-, R⁶-NH-C(=O)- ;
R² à R⁴ :
-(CH₂)₂-, -(CH₂)₃-, -(CH₂)₄-, -CH₂-CH(R⁶)-, -CH₂-CHOR⁷-CH₂-;
R⁶ : un groupe alkyle en C1-C24 ;
R⁷ : l'hydrogène, un groupe alkyle en C1-C24, R⁶-C(=O)-, R⁶-NH-C(=O)- ;
A : -C(=O)-O, -C(=O)-B-C(=O)-O-,
-C(=O)-NH-B-NH-C(=O)-O ;
B : -(CH₂)ₜ-, arylène, éventuellement substitué
n : 1 à 1000 ;
s : 0 à 1000 ;
t : 1 à 12 ;
u : 1 à 5000 ;
v : 0 à 5000 ;
w : 0 à 5000 ;
x : 0 à 5000 ;
y : 0 à 5000 ;
z : 0 à 5000 ;
et
c) le cas échéant un ou plusieurs autres monomères copolymérisables,
suivie d'une saponification au moins partielle des fonctions ester des monomères initiaux a).

3. Utilisation de polymères selon la revendication 2, **caractérisée par le fait que** ces polymères sont obtenus par polymérisation radicalaire de
a) au moins un ester vinylique d'acide carboxylique en C1-C24 en présence de
b) des composés contenant des polyéthers de formule générale I à un poids moléculaire moyen (moyenne en nombre) de 300 à 100 000, les symboles de la formule ayant, indépendamment les uns des autres, les significations suivantes :
R¹ : l'hydrogène, un groupe alkyle en C1-C12, R⁶-C(=O)-, R⁶-NH-C(=O), un radical de polyalcool ;
R⁵ : l'hydrogène, un groupe alkyle en C1-C12, R⁶-C(=O)-, R⁶-NH-C(=O)- ;
R² à R⁴ :
-(CH₂)₂-, -(CH₂)₃-, -(CH₂)₄-, -CH₂-CH(R⁶)-, -CH₂-CHOR⁷-CH₂- ;
R⁶ : un groupe alkyle en C1-C12 ;
R⁷ : l'hydrogène, un groupe alkyle en C1-C12, R⁶-C(=O)-, R⁶-NH-C(=O)- ;
n : 1 à 8 ;
s : 0 ;
u : 2 à 2000 ;
v : 0 à 2000 ;
w : 0 à 2000 ;
et
c) le cas échéant un ou plusieurs autres monomères copolymérisables,
suivie d'une saponification au moins partielle des fonctions ester des monomères initiaux a).

4. Utilisation de polymères selon la revendication 2, **caractérisée par le fait que** ces polymères sont obtenus par polymérisation radicalaire de
a) au moins un ester vinylique d'acide carboxylique en C1-C24 en présence de
b) des composés contenant des polyéthers, de formule générale I, à un poids moléculaire moyen (moyenne en nombre) de 500 à 50 000, les symboles de la formule ayant, indépendamment les uns des autres, les significations suivantes :
R¹ : l'hydrogène, un groupe alkyle en C1-C6, R⁶-C(=O)-, R⁶-NH-C(=O)- ;
R⁵ : l'hydrogène, un groupe alkyle en C1-C6, R⁶-(=O)-, R⁶-NH-C(=O)- ;
R² à R⁴ :
-(CH₂)₂-, -(CH₂)₃-, -(CH₂)₄-, -CH₂-CH(R⁶)-, -CH₂-CHOR⁷-CH₂- ;
R⁶ : un groupe alkyle en C1-C6 ;
R⁷ : l'hydrogène, un groupe alkyle en C1-C6, R⁶-(=O)-, R⁶-NH-C(=O)- ;
n : 1 ;
s : 0 ;
u : 5 à 500 ;
v : 0 à 500 ;
w : 0 à 500 ;
et
c) le cas échéant un ou plusieurs autres monomères copolymérisables,
suivie d'une saponification au moins partielle des fonctions ester des monomères initiaux a), dans des compositions cosmétiques capillaires.

5. Utilisation de polymères selon la revendication 1, **caractérisée par le fait que** les polymères sont obtenus par polymérisation radicalaire de
a) au moins un ester vinylique d'acide carboxylique en C1-C24 en présence de
b) des dérivés de silicones contenant des polyéthers et
c) le cas échéant, un ou plusieurs autres monomères
copolymérisables, suivie d'une saponification au moins partielle des fonctions ester des monomères initiaux a).

6. Utilisation de polymères selon la revendication 5, **caractérisée par le fait que** ces polymères sont obtenus par polymérisation radicalaire de
a) au moins un ester vinylique d'acide carboxylique en C1-C24 en présence de
b) des dérivés de silicones contenant des polyéthers de formule générale II 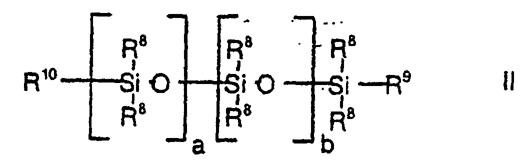
dans laquelle
R⁹ =CH₃ ou 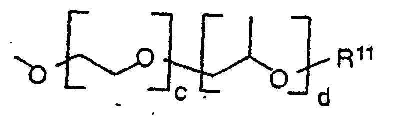 R¹⁰ = CH_{3 ou} R⁹
R¹¹ = H, CH₃, 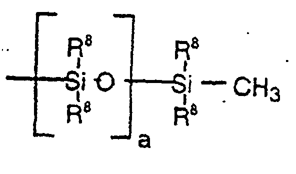 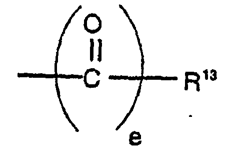 R¹³ représente un radical organique en C1-C40 qui peut contenir des groupes amino, acide carboxylique ou sulfonate, ou bien encore, lorsque e= 0, l'anion d'un acide inorganique,
les groupes R⁸, pouvant être identiques ou différents, appartiennent aux groupes des hydrocarbures aliphatiques en C1-C20, sont des hydrocarbures aliphatiques cycliques en C3-C20, sont de nature aromatique ou sont égaux à R¹², défini par :
R¹² = 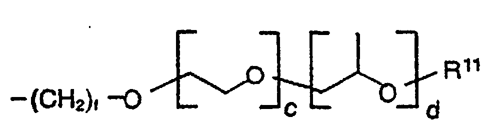 sous réserve que l'un au moins des groupes R⁸, R⁹ ou R¹⁰ est un groupe contenant un oxyde de polyalkylène selon la définition donnée ci-dessus,
et f est un nombre entier allant de 1 à 6,
a et b sont des nombres entiers tels que le poids moléculaire du bloc de polysiloxane se situe entre 300 et 30 000,
c et d sont des nombres entiers qui peuvent aller de 0 à 50, sous réserve que la somme de ce et d est supérieure à 0, et e est égal à 0 ou 1,
et
le cas échéant un ou plusieurs autres monomères copolymérisables, suivie d'une saponification au moins partielle des fonctions ester des monomères initiaux a).

7. Utilisation de polymères selon la revendication 6, **caractérisée par le fait que** la formule II est la suivante :

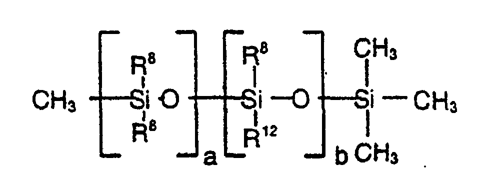

8. Utilisation selon la revendication 1, **caractérisé par le fait que** les polymères sont obtenus par polymérisation radicalaire de
a) au moins un ester vinylique d'acide carboxylique en C1-C24 en présence de
b) des composés contenant des polyéthers obtenus par réaction de polyéthylène-imines avec des oxydes d'alkylène
c) le cas échéant un ou plusieurs autres monomères copolymérisables,
suivie d'une saponification au moins partielle des fonctions ester des monomères initiaux a).

9. Utilisation de polymères selon la revendication 8, **caractérisée par le fait que** les oxydes d'alkylène utilisés sont l'oxyde d'éthylène, l'oxyde de propylène, l'oxyde de butylène ou leurs mélanges.

10. Utilisation de polymères selon les revendications 8 et 9, **caractérisée par le fait que** l'oxyde d'alkylène utilisé est l'oxyde d'éthylène.

11. Utilisation de polymères selon les revendications 8, 9 et 10, **caractérisée par le fait que** la polyéthylène-imine a un poids moléculaire de 300 à 20 000.

12. Utilisation de polymères selon la revendication 1, **caractérisée par le fait que** les composés contenant des polyéthers b) ont été préparés par polymérisation de monomères à insaturation éthylénique contenant des oxydes d'alkylène et le cas échéant d'autres monomères copolymérisables.

13. Utilisation de polymères selon la revendication 12, **caractérisée par le fait que** les composés contenant des polyéthers b) ont été préparés par polymérisation d'éthers vinyliques d'oxydes de polyalkylène et le cas échéant d'autres monomères copolymérisables.

14. Utilisation de polymères selon la revendication 12, **caractérisée par le fait que** les composés contenant des polyéthers b) ont été préparés par polymérisation de (méth) acrylates d'oxydes de polyalkylène et le cas échéant d'autres monomères copolymérisables.

15. Utilisation de polymères selon les revendications 1 à 14, **caractérisée par le fait que** le composant c) est choisi dans le groupe suivant :
acide acrylique, acide méthacrylique, acide maléique, acide fumarique, acide crotonique, anhydride maléique et son hémi-ester, acrylate de méthyle, méthacrylate de méthyle, acrylate d'éthyle, méthacrylate d'éthyle, acrylate de n-butyle, méthacrylate de n-butyle, acrylate de tert-butyle, méthacrylate de tert-butyle, acrylate d'isobutyle, méthacrylate d'isobutyle, acrylate de 2-éthylhexyle, acrylate de stéaryle, méthacrylate de stéaryle, N-tert-butylacrylamide, N-octylacrylamide, acrylate de 2-hydroxyéthyle, acrylates d'hydroxypropyle, méthacrylate de 2-hydroxyéthyle, méthacrylates d'hydroxypropyle, (méth)acrylates d'alkylèneglycols, styrène, acides sulfoniques insaturés, par exemple acide acrylamidopropanesulfonique, vinylpyrrolidone, vinylcaprolactame, éthers vinyliques (par exemple oxyde de méthyle, d'éthyle, de butyle ou de dodécyle et de vinyle), vinylformamide, vinylméthylacétamide, vinylamine, 1-vinylimidazole, 1-vinyl-2-méthylimidazole, méthacrylate de N,N-diméthylaminométhyle et N-[3-(diméthylamino)propyl]méthacrylamide ; chlorure de 3-méthyl-1-vinylimidazolium, méthylsulfate de 3-méthyl-1-vinylimidazolium, méthacrylate de N,N-diméthylaminoéthyle, N-[3-(diméthylamino)propyl]méthacrylamide quaternisé par le chlorure de méthyle, le sulfate de méthyle ou le sulfate de diéthyle.

16. Utilisation de polymères selon les revendications 1 à 15, **caractérisée par le fait que** les proportions mutuelles sont de
a) 10 à 90 % en poids
b) 2 à 90 % en poids
c) 0 à 50 % en poids

17. Utilisation de polymères selon les revendications 1 à 15, **caractérisée par le fait que** les proportions mutuelles sont de
a) 50 à 97 % en poids
b) 3 à 50 % en poids
c) 0 à 30 % en poids

18. Utilisation de polymères selon les revendications 1 à 15, **caractérisée par le fait que** les proportions mutuelles sont de
a) 60 à 97 % en poids
b) 3 à 40 % en poids
c) 0 à 20 % en poids

19. Utilisation selon les revendications 1 à 18, dans laquelle, à la suite de la saponification, on procède à une réticulation.

20. Utilisation selon les revendications 19, dans laquelle la réticulation est réalisée à l'aide d'aldéhydes, de dialdéhydes ou de borates.

21. Compositions cosmétiques capillaires **caractérisées par** la composition suivantes :
a) 0,05 à 20 % en poids du polymère selon la revendication 1
b) 20 à 99,95 % en poids d'eau et/ou d'alcool
c) 0 à 79,05 % en poids d'autres constituants.

22. Compositions cosmétiques capillaires **caractérisées par** la composition suivante :
a) 0,1 à 10 % en poids du polymère selon la revendication 1
b) 20 à 99,9 % en poids d'eau et/ou d'alcool
c) 0 à 70 % en poids d'un agent propulseur,
d) 0 à 20 % en poids d'autres constituants.

23. Compositions cosmétiques capillaires **caractérisées par** la composition suivante :
a) 0,1 à 10 % en poids du polymère selon la revendication 1
b) 55 à 94,8 % en poids d'eau et/ou d'alcool
c) 5 à 20 % en poids d'un agent propulseur
d) 0,1 à 5 % en poids d'un agent émulsionnant
e) 0 à 10 % en poids d'autres constituants.

24. Compositions cosmétiques capillaires **caractérisées par** la composition suivante :
a) 0,1 à 10 % en poids du polymère selon la revendication 1
b) 60 à 99,85 % en poids d'eau et/ou d'alcool
c) 0,05 à 10 % en poids d'un agent gélifiant
d) 0 à 20 % en poids d'autres constituants.

25. Compositions cosmétiques capillaires **caractérisées par** la composition suivante :
a) 0,05 à 10 % en poids du polymère selon la revendication 1
b) 25 à 94,95 % en poids d'eau
c) 5 à 50 % d'agents tensio-actifs
d) 0 à 5 % en poids d'un autre agent conditionnant
e) 0 à 10 % en poids d'autres constituants cosmétiques.

26. Polymères obtenus par polymérisation radicalaire de
a) au moins un ester vinylique d'acide carboxylique en C1-C24 en présence de
b) des dérivés de silicones contenant des polyéthers et
c) le cas échéant un ou plusieurs autres monomères copolymérisables, suivie d'une saponification au moins partielle des fonctions ester des monomères initiaux a).

27. Polymères obtenus par polymérisation radicalaire de
a) un ester vinylique d'acide carboxylique en C1-C24 en présence de
b) des composés contenant des polyéthers obtenus par réaction de polyéthylène-imines avec des oxydes d'alkylène et
c) le cas échéant un ou plusieurs autres monomères copolymérisables,
suivie d'une saponification au moins partielle des fonctions ester des monomères initiaux a).

28. Polymères obtenus par polymérisation radicalaire de
a) un ester vinylique d'acide carboxylique en C1-C24 en présence de
b) des homo- et co-polymères de composés à insaturation éthylénique contenant des polyéthers et
c) le cas échéant, un ou plusieurs autres monomères copolymérisables,
suivie d'une saponification au moins partielle des fonctions ester des monomères initiaux a).

29. Polymères réticulés obtenus par polymérisation radicalaire de
a) au moins un ester vinylique d'acide carboxylique en C1-C24 en présence de
b) des composés contenant des polyéthers et
c) le cas échéant un ou plusieurs autres monomères copolymérisables,
suivie d'une saponification au moins partielle des fonctions ester des monomères initiaux a), l'agent réticulant utilisé étant déjà présent au cours de polymérisation ou étant ajouté à la suite de la polymérisation et de la saponification.

30. Polymères réticulés selon la revendication 29, pour lesquels les agents réticulants utilisés sont des aldéhydes, des dialdéhydes ou des borates.

31. Polymères réticulés selon la revendication 29, **caractérisés par le fait que** l'agent réticulant est déjà présent à la polymérisation.
